(19) Europäisches Patentamt
European Patent Office
Office européen des brevets

(11) EP 0 532 916 B1

(12) **EUROPÄISCHE PATENTSCHRIFT**

(45) Veröffentlichungstag und Bekanntmachung des Hinweises auf die Patenterteilung:
**05.11.1997 Patentblatt 1997/45**

(51) Int. Cl.$^6$: **C07D 241/16**, C09K 19/34, G02F 1/13

(21) Anmeldenummer: **92113901.0**

(22) Anmeldetag: **14.08.1992**

(54) **2-Fluorpyrazine, Verfahren zu ihrer Herstellung und ihre Verwendung in flüssigkristallinen Mischungen**

2-Fluoropyrazines, process for their preparation, and their use in liquid crystalline mixtures

2-Fluoropyrazines, procédé pour leur préparation et leur utilisation dans des mélanges de cristaux liquides

(84) Benannte Vertragsstaaten:
**CH DE FR GB IT LI NL**

(30) Priorität: **17.08.1991 DE 4127306**

(43) Veröffentlichungstag der Anmeldung:
**24.03.1993 Patentblatt 1993/12**

(73) Patentinhaber:
**HOECHST AKTIENGESELLSCHAFT**
**65926 Frankfurt am Main (DE)**

(72) Erfinder:
- **Schlosser, Hubert, Dr.**
  **W-6246 Glashütten/Taunus (DE)**
- **Illian, Gerhard, Dr.**
  **Nerimaku Tokyo 176 (JP)**
- **Kaltbeitzel, Anke, Dr.**
  **W-6090 Rüsselsheim (DE)**
- **Wingen, Rainer, Dr.**
  **W-6234 Hattersheim/Main (DE)**

(56) Entgegenhaltungen:

| | |
|---|---|
| **EP-A- 0 158 137** | **EP-A- 0 284 093** |
| **EP-A- 0 394 464** | **WO-A-90/15116** |
| **WO-A-91/04248** | **WO-A-91/04249** |
| **DE-A- 4 029 165** | **GB-A- 2 175 409** |

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.14.22/3.4

## Beschreibung

Die ungewöhnliche Kombination von anisotropem und fluidem Verhalten der Flüssigkristalle hat zu ihrer Verwendung in elektrooptischen Schalt- und Anzeigevorrichtungen geführt. Dabei können ihre elektrischen, magnetischen, elastischen und/oder thermischen Eigenschaften zu Orientierungsänderungen genutzt werden. Optische Effekte lassen sich beispielsweise mit Hilfe der Doppelbrechung, der Einlagerung dichroitisch absorbierender Farbstoffmolekühle ("guest-host mode") oder der Lichtstreuung erzielen.

Zur Erfüllung der ständig steigenden Praxisanforderungen auf den verschiedenen Anwendungsgebieten besteht laufend ein Bedarf an neuen verbesserten Flüssigkristall ("liquid crystal")-Mischungen und somit auch an einer Vielzahl mesogener Verbindungen unterschiedlicher Struktur. Dies gilt sowohl für die Gebiete, bei denen nematische LC-Phasen verwendet werden, als auch für solche mit smektischen LC-Phasen.

Auf besonderes Interesse sind in den letzten Jahren ferroelektrische flüssigkristalline Mischungen (FLC-Mischungen) gestoßen (siehe z.B. Lagerwall et al. "Ferroelectric Liquid Crystals for Displays", SID Symposium, October Meeting 1985, San Diego, Ca. USA).

Für die praktische Verwendung von ferroelektrischen Flüssigkristallen in elektrooptischen Anzeigen werden chirale, geneigt-smektische Phasen, wie $S_C$*-Phasen,benötigt [siehe R.B. Meyer, L. Liebert, L. Strzelecki und P. Keller, J. Physique 36, L-69 (1975)], die über einen großen Temperaturbereich stabil sind. Dieses Ziel kann man mit Verbindungen erreichen, die selbst solche Phasen, z.B. $S_c$*-Phasen, ausbilden oder aber, indem man nicht chirale geneigt-smektische Phasen ausbildende Verbindungen mit optisch aktiven Verbindungen dotiert [siehe M. Brunet, Cl. Williams, Ann. Phys. 3, 237 (1978)].

Bei der Verwendung ferroelektrischer Flüssigkristallmischungen in elektrooptischen Bauelementen ist eine einheitliche planare Orientierung der Flüssigkristalle notwendig, um ein hohes Kontrastverhältnis zu erzielen. Es hat sich gezeigt, daß sich eine einheitliche planare Orientierung in der $S_C$-Phase erreichen läßt, wenn die Phasenfolge der Flüssigkristallmischung mit abnehmender Temperatur lautet: Isotrop→nematisch→smektisch A→smektisch C (siehe z.B. K. Flatischler et al., Mol. Cryst. Liq. Cryst. 131, 21 (1985); T. Matsumoto et al., p. 468-470, Proc. of the 6th Int. Display Research Conf., Japan Display, 30. September - 2. October 1986, Tokyo, Japan; M. Murakami et al., ibid. p. 344-347).

Für ferroelektrische (chirale smektische) Flüssigkristallmischungen muß zusätzlich die Bedingung erfüllt sein, daß die Ganghöhe (pitch) der Helix in der $S^*_C$-Phase groß, d.h. größer als 5 µm, und in der N*-Phase sehr groß, d.h. größer als 10 µm, bzw. unendlich ist.

Die optische Schaltzeit $\tau$[µs] ferroelektrischer Flüssigkristallsysteme, die möglichst kurz sein soll, hängt von der Rotationsviskosität des Systems $\gamma$[mPas], der spontanen Polarisation $P_S$[nC/cm$^2$] und der elektrischen Feldstärke E[V/m] ab nach der Beziehung

$$\tau \approx \frac{\gamma}{P_s \cdot E}$$

Da die Feldstärke E durch den Elektrodenabstand im elektrooptischen Bauteil und durch die angelegte Spannung festgelegt ist, muß das ferroelektrische Anzeigemedium niedrigviskos sein und eine hohe spontane Polarisation aufweisen, damit eine kurze Schaltzeit erreicht wird.

Schließlich wird neben thermischer, chemischer und photochemischer Stabilität eine kleine optische Anisotropie $\Delta n$, vorzugsweise $\approx$0,13, und eine geringe positive oder vorzugsweise negative dielektrische Anisotropie $\Delta\varepsilon$ verlangt (siehe S.T. Lagerwall et al., "Ferroelectric Liquid Crystals for Displays" SID Symposium, Oct. Meeting 1985, San Diego, Ca, USA).

Die Gesamtheit dieser Forderungen ist im allgemeinen nur mit Mischungen aus mehreren Komponenten zu erfüllen. Als Basis (oder Matrix) dienen dabei bevorzugt Verbindungen, die möglichst selbst bereits die gewünschte Phasenfolge $I \rightarrow N \rightarrow S_A \rightarrow S_C$ aufweisen. Weitere Komponenten der Mischung werden oftmals zur Schmelzpunktserniedrigung und zur Verbreiterung der $S_C$- und meist auch N-Phase, zum Induzieren der optischen Aktivität, zur Pitch-Kompensation und zur Anpassung der optischen und dielektrischen Anisotropie zugesetzt, wobei aber beispielsweise die Rotationsviskosität möglichst nicht vergrößert werden soll.

Einzelne dieser Komponenten und auch bestimmte Mischungen sind bereits aus dem Stand der Technik bekannt. Da aber die Entwicklung, insbesondere von ferrolektrischen Flüssigkristallmischungen, noch in keiner Weise als abgeschlossen betrachtet werden kann, sind die Hersteller von Displays an unterschiedlichen Mischungen interessiert. Dieses u.a. auch deshalb, weil erst das Zusammenwirken der flüssigkristallinen Mischungen mit den einzelnen Bauteilen der Anzeigevorrichtung bzw. der Zellen (z.B. der Orientierungsschicht) Rückschlüsse auf die Qualität auch der flüssigkristallinen Mischungen zuläßt.

In EP-B 0 158 137 werden 4-Fluorpyrimidine als Verbindungen und als Mischungskomponenten allgemein beschrieben. Sie weisen jedoch keine oder nur eine geringe Tendenz zur Ausbildung smektischer Phasen auf.

In DE-A 40 29 165 sowie in werden 4-Fluorpyrimidine als

In WO-A-9 104 249 werden Fluorpyridine als Komponenten für Flüssigkristallmischungen vorgestellt Komponenten für ferroelektrische Flüssigkristallmischungen vorgestellt.

Desweiteren ist bekannt, daß Mono- und Difluorphenylverbindungen als Komponenten von Flüssigkristallmischungen verwendet werden können (JP-A 2169-537; V. Reiffenrath, The Twelfth International Liquid Crystal Conference, Freiburg, 15.-19. August 1988). Diese Verbindungen haben jedoch zum Teil keine $S_C$-Phase. Desweiteren treten aufgrund fluorophober Wechselwirkungen häufig Mischbarkeitsprobleme mit strukturell unterschiedlichen Mischungskomponenten, z.B. Phenylpyrimidinen, auf.

Pyrazinderivate zeigen ebenfalls flüssigkristallines Verhalten unter Ausbildung einer $S_C$-Phase (D. Demus, H. Demus, H. Zaschke, Flüssige Kristalle in Tabellen, VEB Deutscher Verlag für Grundstoffindustrie, Leipzig 1974; JP-61129-171-A; GB-A-2 175 409). Eine bei diesen Verbindungen häufig auftretende $S_B$-Phase und hohe Phasenübergangstemperaturen beeinträchtigen jedoch deren Verwendung in Flüssigkristallmischungen.

Die vorliegende Erfindung betrifft neue 2-Fluorpyrazin-Derivate und deren Verwendung als Komponenten für Flüssigkristallmischungen, insbesondere für ferroelektrische, wobei man mindestens ein 2-Fluorpyrazin der allgemeinen Formel (I) als Komponente in einer Flüssigkristallmischung einsetzt.

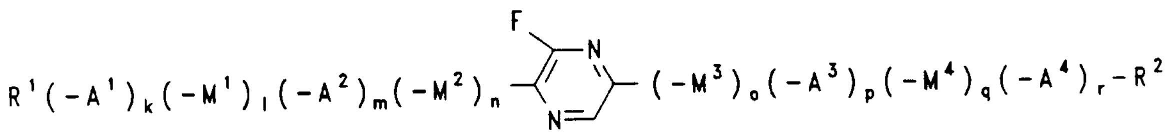

(I)

Die Symbole haben hierbei folgende Bedeutung:

$R^1$, $R^2$ unabhängig voneinander H, F, Cl, CN, NCS, $-CF_3$, $-OCF_3$, $-OCHF_2$ oder geradkettiges oder verzweigtes Alkyl mit 1 bis 6 C-Atomen (mit oder ohne asymmetrischem C-Atom), wobei auch eine oder zwei nichtbenachbarte $-CH_2$-Gruppen jeweils durch -O-, -S-, -CO-, -CO-O-, -O-CO-, -CO-S-, -S-CO-, -O-CO-O-, -CH=CH-, -C≡C-,

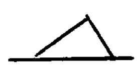

oder $-Si(CH_3)_2-$ ersetzt sein können und wobei auch ein oder mehrere H-Atome des Alkylrestes jeweils durch F, Cl, Br und/oder CN substituiert sein können, oder eine der nachfolgenden chiralen Gruppen:

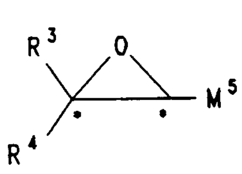, 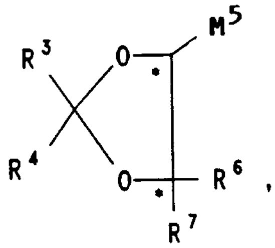, 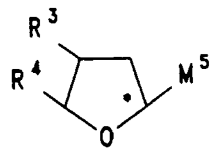, 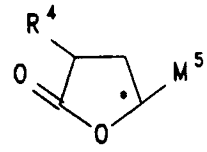,

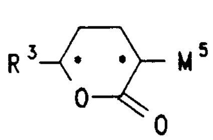, 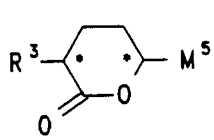, 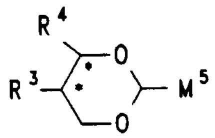, 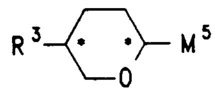,

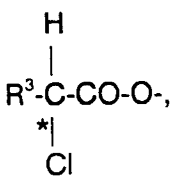 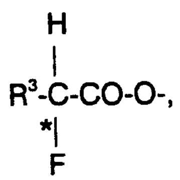 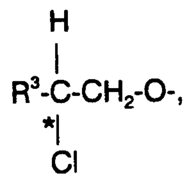 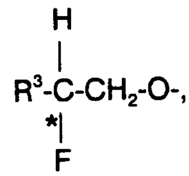

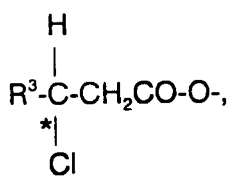 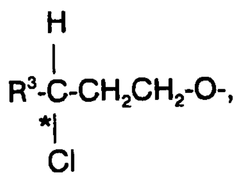

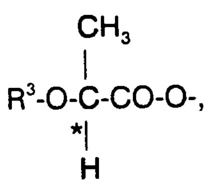 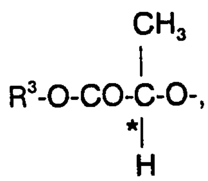

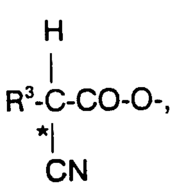 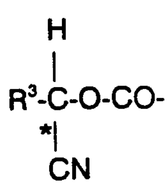

$A^1$, $A^2$, $A^3$, $A^4$ gleich oder verschieden 1,4-Phenylen, Pyrazin-2,5-diyl, Pyridazin-3,6-diyl, Pyridin-2,5-diyl, Pyrimidin-2,5-diyl, wobei ein oder zwei H-Atome durch F, Cl und/oder CN ersetzt sein können, trans-1,4-Cyclohexylen, bei dem ein oder zwei H-Atome durch CN und/oder $CH_3$ ersetzt sein können, (1,3,4)-Thiadiazol-2,5-diyl, 1,3-Dioxan-2,5-diyl, Naphthalin-2,6-diyl, Bicyclo[2.2.2.]octan-1,4-diyl oder 1,3-Dioxaborinan-2,5-diyl;
$M^1$, $M^2$, $M^3$, $M^4$ gleich oder verschieden -O-, -S-, -CO-, -CO-O-, -O-CO-, -CO-S-, -S-CO-, -O-CO-O-, $-CH_2-O-$, $-O-CH_2-$, $-CH_2-CH_2-$, -CH=CH- oder -C≡C-;
$R^3$, $R^4$, $R^6$, $R^7$ unabhängig voneinander H oder geradkettiges oder verzweigtes Alkyl mit 1 bis 16 C-Atomen; oder $R^3$ und $R^4$ zusammen auch $-(CH_2)_4-$ oder $-(CH_2)_5-$, wenn als Substituenten an ein Dioxolan-System gebunden;
$M^5$ $-CH_2-O-$, -CO-O-, $-O-CH_2-$, -O-CO- oder eine Einfachbindung;
k, l, m, n, o, p, q, r Null oder 1, unter der Bedingung, daß die Summe k+m+p+r kleiner 4 und größer Null ist.

In einer bevorzugten Ausführung der Erfindung haben die Symbole folgende Bedeutung:

$R^1$, $R^2$ unabhängig voneinander H, F, CN, oder geradkettiges oder verzweigtes Alkyl mit 1 bis 16 C-Atomen (mit oder ohne asymmetrischem C-Atom), wobei auch eine $-CH_2$-Gruppe durch -O-, -S-, -CO-, -CO-O-, -O-CO-, -O-CO-O-, -CH=CH-, -C≡C-,

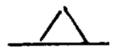

oder $-Si(CH_3)_2$- ersetzt sein kann, oder eine der nachfolgenden chiralen Gruppen:

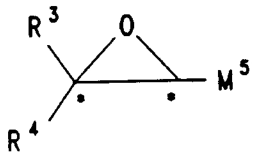 ,
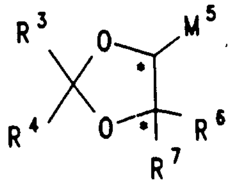 ,
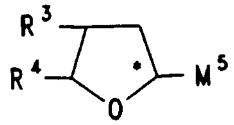 ,
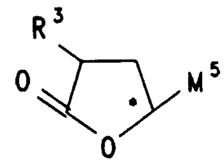 ,

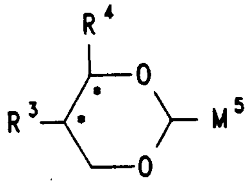 ,

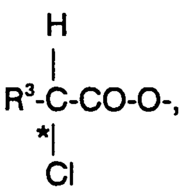
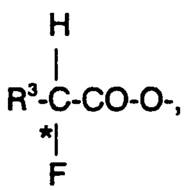
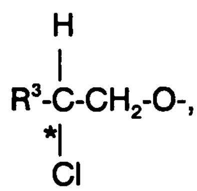
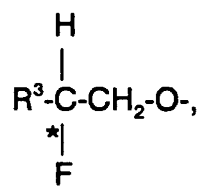

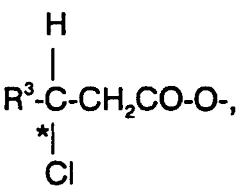
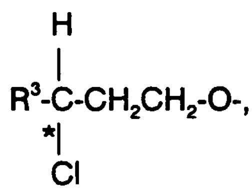

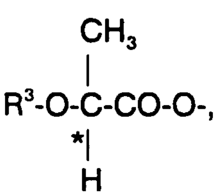
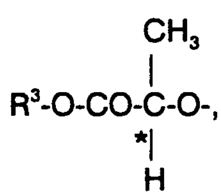

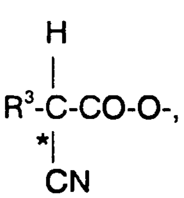
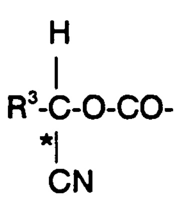

$A^1$, $A^2$` $A^3$, $A^4$ gleich oder verschieden 1,4-Phenylen, Pyrazin-2,5-diyl, Pyridazin-3,6-diyl, Pyridin-2,5-diyl, Pyrimidin-2,5-diyl, wobei ein oder zwei H-Atome jeweils durch F ersetzt sein können, trans-1,4-Cyclohexylen, (1,3,4)-Thiadiazol-2,5-diyl oder Naphthalin-2,6-diyl, 1,3-Dioxan-2,5-diyl oder Bicyclo[2.2.2.]octan-1,4-diyl;
$M^1$, $M^2$, $M^3$, $M^4$ gleich oder verschieden -O-, -CO-, -CO-O-, -O-CO-, $-CH_2-O-$, $-O-CH_2-$, $-CH_2-CH_2-$, -CH=CH- oder -C≡C-;
$R^3$, $R^4$, $R^6$, $R^7$ unabhängig voneinander H oder geradkettiges oder verzweigtes Alkyl mit 1 bis 16 C-Atomen; $R^3$ und $R^4$ zusammen auch $-(CH_2)_4-$ oder $-(CH_2)_5-$ wenn als Substituenten an einem Dioxolan-System gebunden;
$M^5$ $-CH_2-O-$, -CO-O-, $-O-CH_2-$, -O-CO- oder eine Einfachbindung.

Besonders bevorzugt sind 2-Fluorpyrazin-Derivate, bei denen die Symbole folgende Bedeutung haben:

$R^1$, $R^2$ unabhängig voneinander H, F, CN oder geradkettiges oder verzweigtes Alkyl mit 1 bis 16 C-Atomen (mit oder ohne asymmetrischem C-Atom), wobei auch eine $-CH_2$-Gruppe durch -O-, -CO-, -CO-O-, -O-CO-, -CH=CH-, oder $-Si(CH_3)_2$-ersetzt sein kann, oder eine der nachfolgenden chiralen Gruppen:

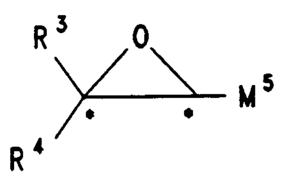 , 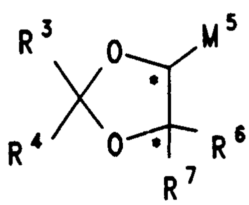 , 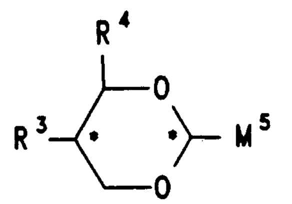 ,

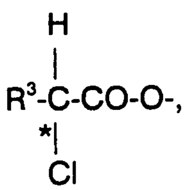 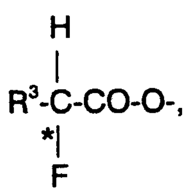

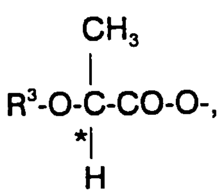 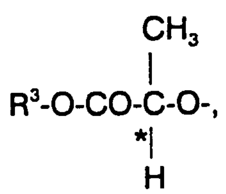

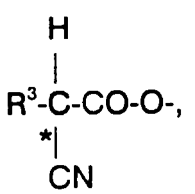 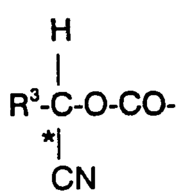

$A^1$, $A^2$, $A^3$, $A^4$ gleich oder verschieden 1,4-Phenylen, Pyrazin-2,5-diyl, Pyridazin-3,6-diyl, Pyridin-2,5-diyl, Pyrimidin-2,5-diyl, bei dem ein H-Atom durch F ersetzt sein kann, trans-1,4-Cyclohexylen, Naphthalin-2,6-diyl oder 1,3-Dioxan-2,5-diyl;
$M^1$, $M^2$, $M^3$, $M^4$ gleich oder verschieden -O-, -CO-O-, -O-CO-, $-O-CH_2-$, $-CH_2-O-$, $-CH_2-CH_2-$ oder -CH=CH-;
$R^3$, $R^4$, $R^6$, $R^7$ unabhängig voneinander H oder geradkettiges oder verzweigtes Alkyl mit 1 bis 16 C-Atomen; $R^3$ und $R^4$ zusammen auch $-(CH_2)_4-$ oder $-(CH_2)_5$)- wenn als Substituenten an ein Dioxolan-System gebunden;
$M^5$ $-CH_2-O-$, -CO-O-, $-O-CH_2-$, -O-CO- oder eine Einfachbindung.

Insbesondere bevorzugt ist ein 2-Fluorpyrazin, bei dem

$R^1$, $R^2$ unabhängig voneinander H oder Alkyl mit 1 bis 16 C-Atomen ist, wobei eine $-CH_2$-Gruppe durch -O-, ersetzt sein kann, oder die chirale Gruppe

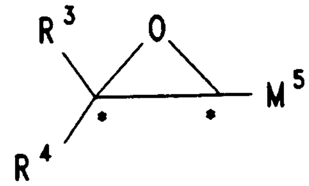

$A^1$, $A^2$, $A^3$, $A^4$ gleich oder verschieden 1,4-Phenylen, Pyrimidin-2,5-diyl, 1,4-Cyclohexylen bedeutet,
$M^1$, $M^2$, $M^3$, $M^4$ gleich oder verschieden -O-, -COO-, $-CH_2-O-$, $-O-CH_2-$, $M^5$ gleich $-CH_2-O-$ oder -COO- ist und
$R^3$, $R^4$ unabhängig voneinander H oder geradkettiges Alkyl mit 1 bis 10 C-Atomen

ist.

Die 2-Fluorpyrazine sind chemisch und photochemisch stabil. Im Gegensatz zu den bereits bekannten Pyrazinderivaten (s.o.) besitzen die erfindungsgemäßen Verbindungen niedrige Phasenübergangstemperaturen, die ihre Verwendung in Flüssigkristallmischungen begünstigen. Sie weisen zum Teil breite $S_A$ Phasen aufweisen, so daß durch Zugabe dieser Verbindungen zu flüssigkristallinen Mischungen der Bereich der $S_A$ Phase vergrößert oder erzeugt werden kann. Auf diese Weise kann die Orientierbarkeit der Mischung in einer Zelle verbessert werden. Eine breite $S_A$ Phase kann außerdem tendenziell den Schaltwinkel $\Theta_{eff}$ einer ferroelektrischen Flüssigkristallmischung senken und damit deren Schaltgeschwindigkeit steigern.

Die erfindungsgemäßen Verbindungen können auch über eine $S_C$ und N-Phase verfügen.

Die 2-Fluorpyrazin-Verbindungen sind mit einer Vielzahl anderer Flüssigkristalle zu ferroelektrischen Flüssigkristallmischungen kombinierbar, z.B. mit Phenylpyrimidinen, Phenylpyridinen, Phenylbenzoaten, Naphthalinverbindungen, Biphenylderivaten, Thiadiazolen.

Besonders geeignet sind die erfindungsgemäßen Verbindungen auch für die Abmischung mit Difluorphenyl-, Fluorpyridin- oder Thiadiazol-Verbindungen, da diese ebenfalls ein großes negatives $\Delta\varepsilon$ aufweisen. Auf dieser Basis lassen sich Mischungen erstellen, bei denen eine Rückrelaxation des Direktors nach dem Schalten durch eine AC-Feld-Stabilisierung verhindert werden kann.

Flüssigkristalline Mischungen, die Verbindungen der allgemeinen Formel (I) enthalten, sind besonders für die Verwendung in elektrooptischen Schalt-und Anzeigevorrichtungen (Displays) geeignet. Schalt- und Anzeigevorrichtungen (LC-Displays) weisen u.a. folgende Bestandteile auf: ein flüssigkristallines Medium, Trägerplatten (z.B. aus Glas oder Kunststoff), beschichtet mit transparenten Elektroden, mindestens eine Orientierungsschicht, Abstandshalter, Kleberahmen, Polarisatoren sowie für Farbdisplays dünne Farbfilterschichten. Weitere mögliche Komponenten sind Antireflex-, Passivierungs-, Ausgleichs- und Sperrschichten sowie elektrisch-nichtlineare Elemente, wie Dünnschichttransistoren (TFT) und Metall-Isolator-Metall-(MIM)-Elemente. Im Detail ist der Aufbau von Flüssigkristalldisplays bereits in einschlägigen Monographien beschrieben (z.B. E. Kaneko, "Liquid Crystal TV Displays: Principles and Applications of Liquid Crystal Displays", KTK Scientific Publishers, 1987, Seiten 12-30 und 163-172).

Die Herstellung der erfindungsgemäßen Verbindungen I kann durch in den Schemata 1 bis 4 dargestellte Verfahren erfolgen, wobei ausgehend von 2,6-Dichlorpyrazin (II) durch mehrstufige Umsetzung die Seitenketten $R^1(-A^1)_k(-M^1)_l(-A^2)_m(-M^2)_n$ und $(-M^3)_o(-A^3)_p(-M^4)_q(-A^4)_r-R^2$ in die 3- bzw. 6-Position des Pyrazinringes eingebracht werden.

Ausgangsverbindung dieses Verfahrens ist das kommerziell erhältliche 2,6-Dichlorpyrazin (II), welches durch Umsetzung mit 3 bis 20, insbesondere 5 bis 10 Mol-Äquivalenten eines Fluorid-Reagenzes, wie Silberfluorid, Natriumfluorid, Kaliumfluorid oder Cäsiumfluorid, bei Temperaturen zwischen 50 und 200°C, insbesondere zwischen 100 und 150°C, unter Verwendung katalytischer Mengen (1 bis 20 Mol-Prozent, insbesondere 5 bis 15 Mol-Prozent) mindestens eines Komplexbildners, wie 18-Krone-6, Dibenzo-18-Krone-6 bder 1,10-Diaza-4,7,13,16,21,24-hexaoxabicyclo[8.8.8]-hexacosan, 2,6-Difluorpyrazin (III) liefert.

Der Austausch eines Fluorsubstituenten in (III) gegen eine Gruppierung der allgemeinen Formel $Z^2 = (-M^3)_o(A^3)_p(M^4)_q(A^4)_r-R^2$ durch Umsetzung mit einer Metallverbindung von $Z^2$, z.B. einer Lithium-, Natrium-, Kalium- oder Magnesiumverbindung, bei Temperaturen zwischen -40 und 100°C, insbesondere zwischen -10 und 70°C, in einem inerten Reaktionsmedium, z.B. Diethylether, Tetrahydrofuran, 1,4-Dioxan, Ethylenglykoldiethylether oder Diethylenglykoldiethylether, führt zu Verbindungen der Formel (V).

Durch die Umsetzung von 2,6-Dichlorpyrazin mit 1 bis 3, insbesondere 1,5 bis 2,5 Mol-Äquivalenten eines der oben angegebenen Fluorid-Reagenzien bei Temperaturen zwischen 50 und 200°C, insbesondere zwischen 100 und 150°C, und einem Druck zwischen 50 und 300 mm Hg, insbesondere zwischen 100 und 200 mm Hg, unter Verwendung katalytischer Mengen (1 bis 20 Mol-Prozent, insbesonders 5 bis 15 Mol-Prozent) eines der oben angegebenen Komplexbildner wird 2-Chlor-6-fluorpyrazin (IV) erhalten.

Die Kreuzkupplung von Verbindung (IV) mit metallorganischen Derivaten von $Z^2$, z.B. Grignard-, Lithium- und Zinkderivaten, sowie Boronsäuren von $Z^2$ unter Verwendung von Übergangsmetallkatalysatoren, z.B. Dichloro[1,3-bis(diphenylphosphino)propan]nickel [II] und Tetrakis(triphenylphosphin)palladium[0], bei Temperaturen zwischen -40 und 200°C, insbesondere zwischen -10 und 100°C, in Reaktionsmedien, wie Benzol/Ethanol/Wasser für die Umsetzung mit Boronsäuren von $Z^2$ und z.B. Diethylether oder Tetrahydrofuran für die Umsetzung mit Grignard-, Lithium- und Zinkderivaten von $Z^2$, liefert ebenfalls Verbindungen des Typs (V).

2-Fluorpyrazine des Typs (V) können durch Behandlung mit einer Lithiumverbindung, z.B. Lithiumalkylen oder Lithiumamiden, bei Temperaturen zwischen -40 und -100°C, insbesondere zwischen -60 und -80°C, in einem inerten Reaktionsmedium, z.B. Diethylether, Tetrahydrofuran oder Ethylenglykoldiethylether, in 2-Fluor-3-lithiumpyrazine der Formel (VI) überführt werden. 3-Lithiumpyrazine der allgemeinen Formel (VI) sind der Umsetzung mit elektrophilen Verbindungen zugänglich, wodurch entweder direkt oder über weitere Zwischenstufen, wie Verbindungen der Formeln (VII), (VIII), (IX), (X), (XI) und (XII), 2-Fluorpyrazine der Formel (I) erhalten werden können.

So führt die Reaktion von Verbindungen des Typs (VI) mit Nitrilen, Carbonsäurehalogeniden und Formylmethylderivaten von $Z^3$ bei Temperaturen zwischen -40 und -100°C, insbesondere zwischen -60 und -80°C, in einem inerten Reaktionsmedium, z.B. Diethylether, Tetrahydrofuran oder Ethylenglykoldiethylether, direkt zu 2-Fluorpyrazinen der Formel (I). Olefinische 2-Fluorpyrazine (I) lassen sich durch Hydrierung der olefinischen Doppelbindung nach an sich literaturbekannten Methoden (siehe z.B. Houben-Weyl, Methoden der Organischen Chemie, Georg Thieme Verlag, Stuttgart) in gesättigte Spezies (I) umwandeln.

Die Umsetzung von 2-Fluor-3-lithiumpyrazinen (VI) mit Halogenen, z.B. Chlor, Brom oder Jod, bei Temperaturen zwischen -40 und -100°C, insbesondere zwischen -60 und -80°C, in einem inerten Reaktionsmedium, z.B. Diethylether, Tetrahydrofuran oder Ethylenglykoldiethylether, führt zu 2-Fluor-3-halogenpyrazinen der Formel (VII). Durch Kreuzkupplung von Verbindungen des Typs (VII) mit metallorganischen Derivaten von $Z^1$, z.B. Grignard-, Lithium- und Zinkderivaten, sowie Boronsäuren von $Z^1$ unter Verwendung von Übergangsmetallkatalysatoren, z.B. Dichloro[1,3-bis(diphenylphosphino)propan]nickel [II] und Tetrakis(triphenylphenylphosphin)palladium[0], bei Temperaturen zwischen -40 und 200°C, insbesondere zwischen -10 und 100°C, in Reaktionsmedien, wie Benzol/Ethanol/Wasser für die Umsetzung mit Boronsäuren von $Z^1$ und z.B. Diethylether oder Tetrahydrofuran für die Umsetzung mit Grignard-, Lithium-und Zinkderivaten von $Z^1$, erhält man 2-Fluorpyrazine (I).

2-Fluor-3-lithiumpyrazine (VI) führen nach der Behandlung mit Kohlendioxid bei Temperaturen zwischen -40 und -100°C, insbesondere zwischen -60 und -80°C, in einem inerten Reaktionsmedium, z.B. Diethylether, Tetrahydrofuran oder Ethylenglykdldiethylether, zu 2-Fluor-3-pyrazincarbonsäuren der allgemeinen Formel (VIII). Die Spezies (VIII) können nach an sich literaturbekannten Methoden (siehe z.B. Houben-Weyl, Methoden der Organischen Chemie, Georg Thieme Verlag, Stuttgart), entweder direkt durch Veresterung mit Alkoholen von $Z^3$ unter Zuhilfenahme geeigneter Kondensationsmittel, z.B. Carbodiimiden, zu 2-Fluorpyrazinen (I), oder nach Reduktion zu 2-Fluor-3-hydroxymethylpyrazinen (IX) mit geeigneten Reduktionsmitteln, z.B. komplexen Hydriden, durch Veresterung mit Carbonsäuren bzw. Carbonsäurehalogeniden von $Z^3$ oder Veretherung mit Alkoholen bzw. Halogeniden von $Z^3 = R^1(-A^1)_k(-M^2)_l(-A^2)_m$ zu Verbindungen der Formel (I) umgesetzt werden.

Durch Reaktion von 2-Fluor-3-lithiumpyrazinen (VI) mit Ameisensäureamiden bei Temperaturen zwischen -40 und -100°C, insbesondere -60 und -80°C, in einem inerten Reaktionsmedium, z.B. Diethylether, Tetrahydrofuran oder Ethylenglykoldiethylether, werden 2-Fluor-3-formylpyrazine (X) erhalten, welche nach der sauer katalysierten Acetalisierung mit 2-$Z^4$-1,3-Propandiolen nach an sich literaturbekannten Methoden (siehe z.B. Houben-Weyl, Methoden der Organischen Chemie, Georg Thieme Verlag, Stuttgart) 2-Fluorpyrazine des Typs (I) liefern.

Bei der sukzessiven Behandlung der 2-Fluor-3-lithiumpyrazine (VI) mit Borsäuretrialkylestern bei Temperaturen zwischen -40 und -100°C, insbesondere zwischen -60 und -80°C, und wäßriger Säure bei Temperaturen zwischen -10 und 50°C, insbesondere zwischen 10 und 30°C, in einem inerten Reaktionsmedium, z.B. Diethylether, Tetrahydrofuran oder Ethylenglykoldiethylether, werden 2-Fluor-3-pyrazinboronsäuren der Formel (XI) erhalten.

Die Boronsäuren (XI) können Kupplungsreaktionen mit Halogeniden von $Z^1$ unter Verwendung eines Übergangsmetallkatalysators, z.B. Tetrakis(triphenylphosphin)palladium[0] bei Temperaturen zwischen 30 und 200°C, insbesondere zwischen 50 und 100°C, in Reaktionsmedien, wie Benzol/Ethanol/Wasser, zur Darstellung von Verbindungen des Typs (I) unterworfen werden.

2-Fluorpyrazine (I) werden aus den Boronsäuren (XI) desweiteren durch deren Veresterung mit 2-$Z^4$-1,3-Propandiolen ($Z^4 = R^1(-A^1)_k(-M^1)_l$) nach an sich literaturbekannten Methoden (siehe z.B. Houben-Weyl, Methoden der Organischen Chemie, Georg Thieme Verlag, Stuttgart) erhalten.

Die Oxidation der Boronsäuren (XI) mit Peroxiden, z.B. Wasserstoffperoxid, bei Temperaturen zwischen 10 und 100°C, insbesondere zwischen 30 und 70°C, in Reaktionsmedien, wie Diethylether oder Tetrahydrofuran, führt zu den 2-Fluor-3-hydroxypyrazinen (XII), welche sich nach an sich literaturbekannten Methoden (siehe z.B. Houben-Weyl, Methoden der Organischen Chemie, Georg Thieme Verlag, Stuttgart) durch Veresterung mit Carbonsäuren bzw. Carbonsäurehalogeniden von $Z^3$ oder durch Veretherung mit Alkoholen bzw. Halogeniden von $Z^3$ in 2-Fluorpyrazine der allgemeinen Formel (I) überführen lassen.

Schema 1:

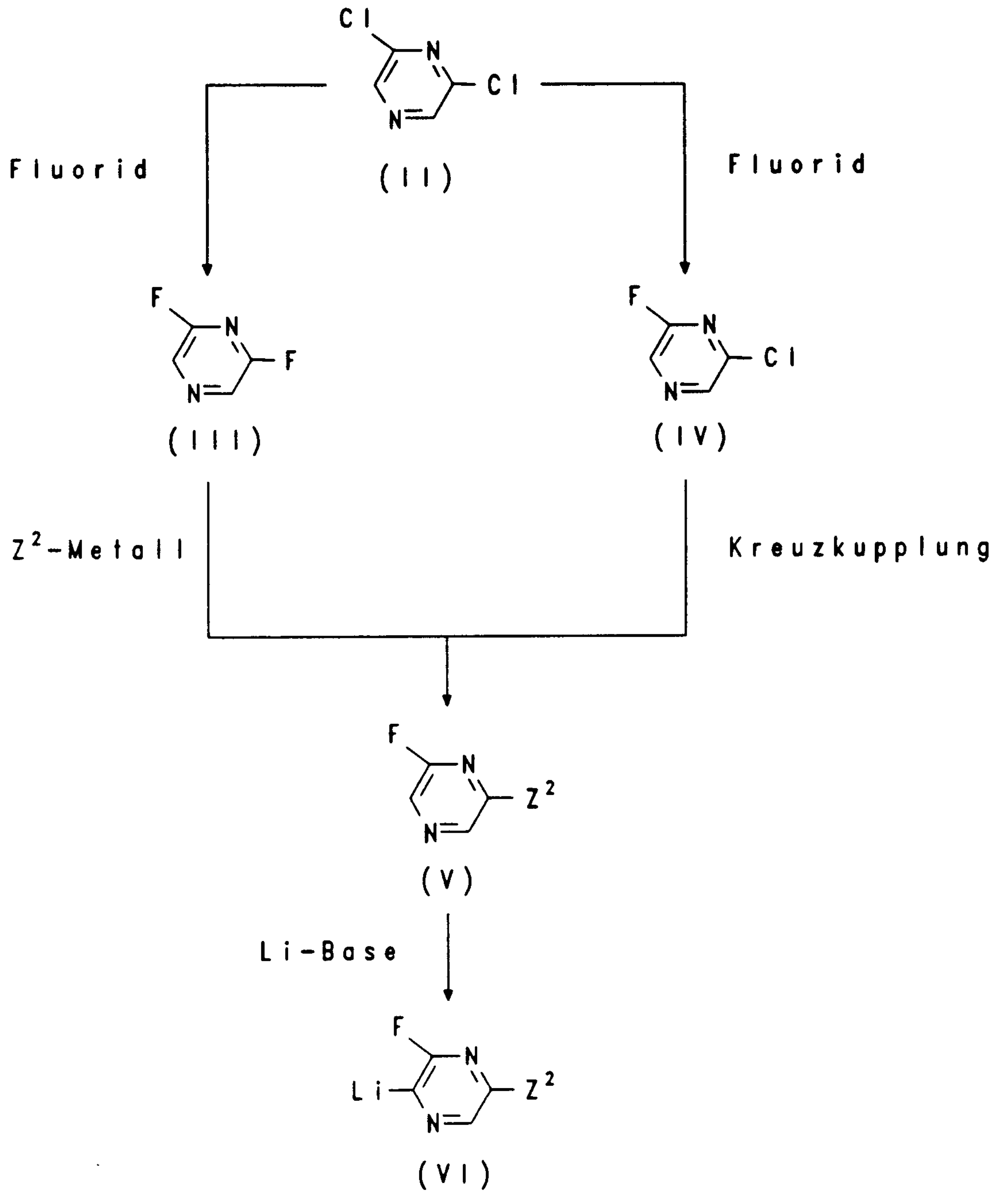


$Z^2 = (-M^3)_o(-A^3)_p(-M^4)_q(-A^4)_r-R^2$

Schema 2:

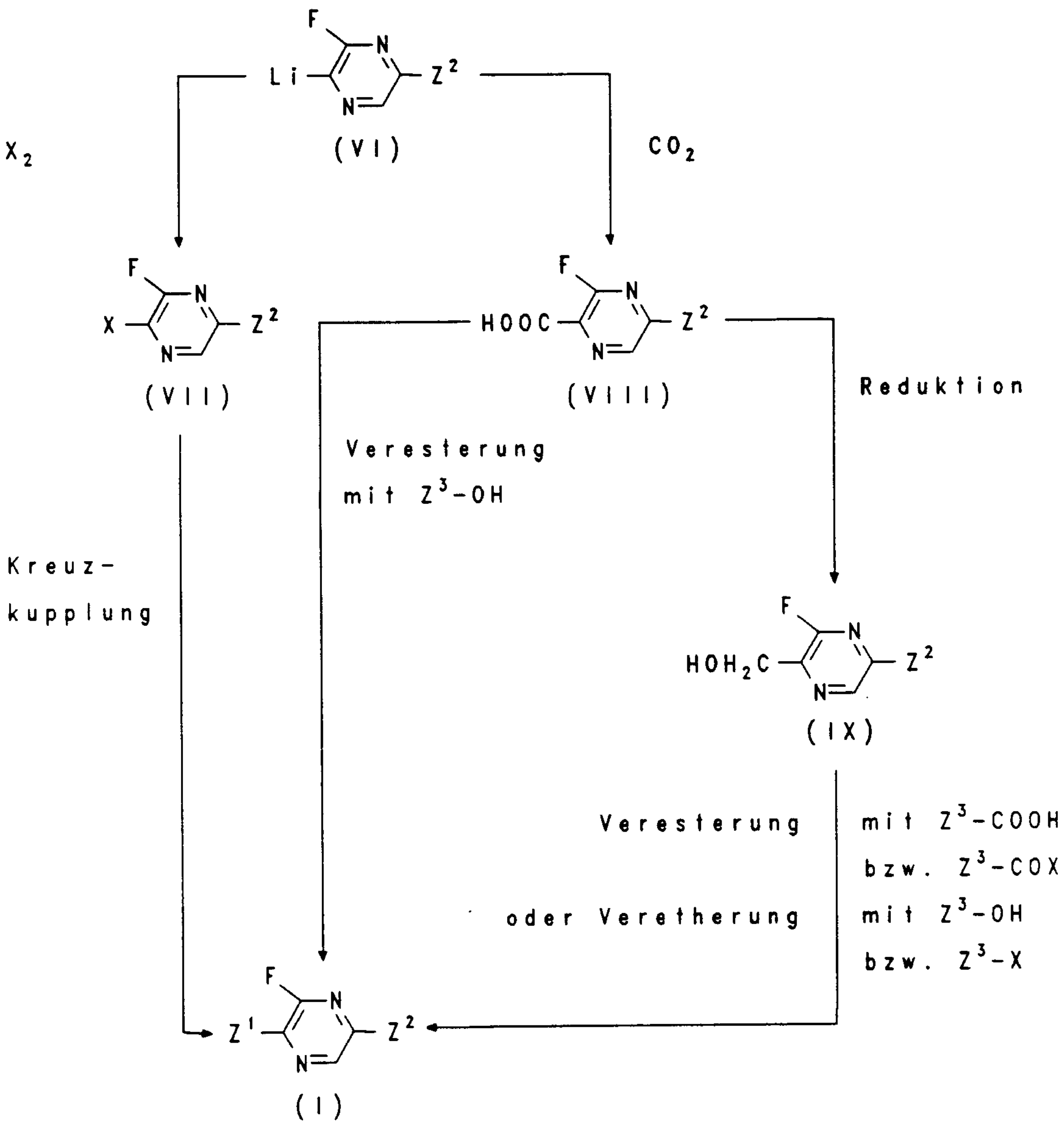


$Z^1 = R^1(-A^1)_k(-M^1)_l(-A^2)_m(M^2)_n$;
$Z^3 = R^1(-A^1)_k(-M^2)_l(-A^2)_m$;
$Z^2$ = siehe Schema 1
X = Cl, Br, I

Schema 4:

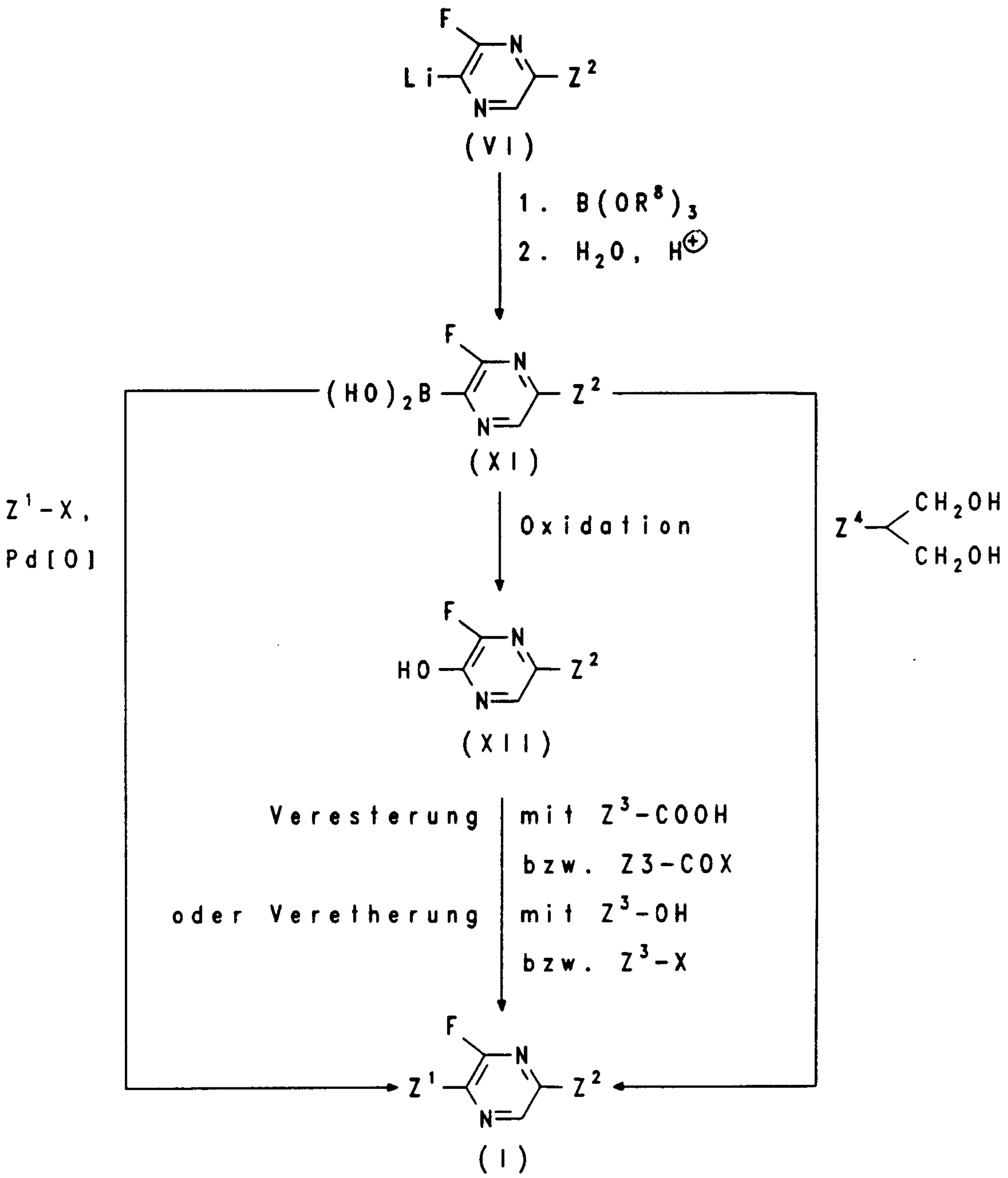


$Z^1$, $Z^2$, $Z^3$, $Z^4$, X, $R^8$ siehe Schemata 1, 2 und 3

Schema 3:

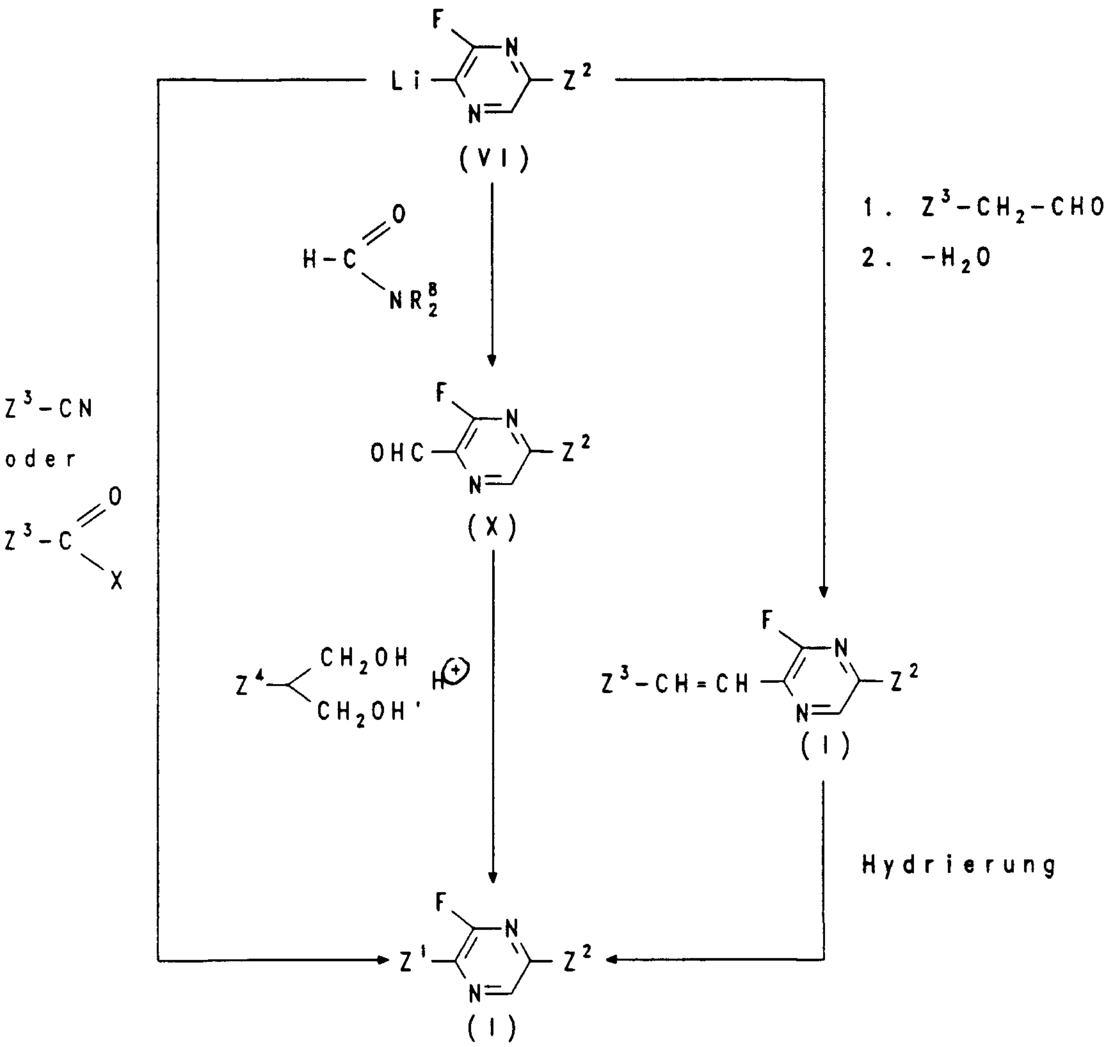


$Z^1$, $Z^2$, $Z^3$, X = siehe Schemata 1 und 2
$Z^4$ = $R^1(-A^1)_k(-M^1)_l$
$R^8$ = geradkettiges oder verzweigtes Alkyl mit 1 bis 16 C-Atomen

Die Erfindung wird durch die nachfolgenden Beispiele näher erläutert:

Für die ferroelektrischen Flüssigkristallmischungen wurden die Werte für die spontane Polarisation $P_s$[nC/cm$^2$], die elektrische Schaltzeit $\tau$[$\mu$s] und die helikale Verdrillungskraft in der nematischen Phase bestimmt, wobei die ersten beiden Messungen bei einer Temperatur von 20°C vorgenommen wurden.

Die $P_s$-Werte wurden nach der Methode von H. Diamant et al. (Rev. Sci. Instr., 28, 30, 1957) gemessen, wobei Meßzellen mit 10 $\mu$m Elektrodenabstand ohne Orientierungsschicht verwendet wurden.

Zur Messung der elektrischen Schaltzeit wurde eine Rechteckspannung von $\pm$ 100 Volt an die oben beschriebene Meßzelle angelegt und der Polarisationsumkehrstrom gemessen. Die elektrische Schaltzeit $T$ ist definiert als Zeitverzögerung zwischen der Spannungsumpolung und dem maximalen Polarisationsstrom.

Die helikale Verdrillung in der nematischen Phase (HTP) wurde nach der Grandjean-Cano Methode mit Hilfe einer Keilzelle bestimmt (F. Grandjean, CR Acad. Sci. (Paris) 172, 71 (1921), R. Cano, Bull. Soc. Franc. Minéral. Crystallogr. XC (333 (1967)).

Die Phasenumwandlungstemperaturen wurden beim Aufheizen mit Hilfe eines Polarisationsmikroskops anhand der Texturänderungen bestimmt. Die Bestimmung des Schmelzpunkts wurde hingegen mit einem DSC-Gerät durchgeführt. Die Angabe der Phasenumwandlungstemperaturen zwischen den Phasen

| | |
|---|---|
| Nematisch | (N bzw. N*) |
| Smektisch-C | ($S_C$ bzw. $S_C$*) |
| Smektisch-A | ($S_A$ bzw. $S_A$*) |
| Kristallin | (X) |

erfolgte in °C, und die Werte stehen zwischen den Phasenbezeichnungen in der Phasenfolge.

Beispiel 1

2-Fluor-3-octyl-6-(4-octyloxyphenyl)pyrazin

1a:

40,00 g (268,5 mmol) 2,6-Dichlorpyrazin, 31,20 g (537,0 mmol) Kaliumfluorid und 7,10 g (26,9 mmol) 18-Krone-6 werden 0,5 h unter Normaldruck und 3 h bei einem Druck von 160 mm Hg auf 120°C erhitzt, wobei eine farblose Flüssigkeit überdestilliert, die nach Reinigung durch Vakuumdestillation bei einem Druck von 160 mm Hg 22,59 g 2-Chlor-6-fluorpyrazin liefert.

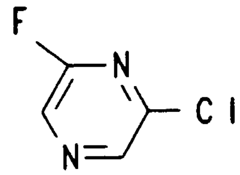

1b:

7,99 g (60,0 mmol) 2-Chlor-6-fluorpyrazin, 15,08 g (60,0 mmol) 4-Octyloxyphenylboronsäure, 12,78 g (120,0 mmol) Natriumcarbonat und 0,72 g (0,6 mmol) Tetrakis(triphenylphosphin)palladium[0] werden in 400 ml Benzol, 300 ml Ethanol und 150 ml Wasser 2 h auf 80°C erhitzt. Nach Verteilen des Reaktionsgemisches zwischen wäßriger Natriumchloridlösung und Ether wird die organische Phase mit wäßriger Natriumchloridlösung gewaschen, über Natriumsulfat getrocknet, filtriert, vom Lösungsmittel befreit und chromatographisch (Kieselgel/Hexan:Ethylacetat = 8:2) gereinigt. Es werden 17,18 g 2-Fluor-6-(4-octyloxyphenyl)pyrazin erhalten.

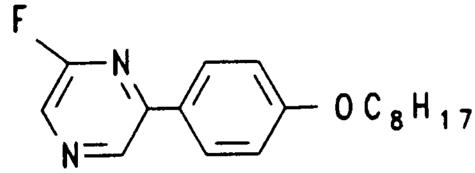

1c:

3,50 g (24,8 mmol) 2,2,6,6-Tetramethylpiperidin und 15,50 ml (24,80 mmol) einer 1,6 M n-Butyllithiumlösung in Hexan werden in 80 ml Tetrahydrofuran 0,5 h bei 0°C gerührt, mit 250 ml Tetrahydrofuran versetzt und auf -78°C abgekühlt. Anschließend werden bei -78°C 5,00 g (16,5 mmol) 2-Fluor-6-(4-octyloxyphenyl)pyrazin in 150 ml Tetrahydrofuran und nach einstündigem Rühren 0,85 ml Brom zugetropft. Das Reaktionsgemisch wird über Nacht gerührt, wobei es sich auf Raumtemperatur erwärmt, zwischen Ether und wäßriger Natriumchloridlösung verteilt, die organische Phase mit wäßriger Natriumchloridlösung gewaschen, über Natriumsulfat getrocknet, filtriert, vom Lösungsmittel befreit und chromatographisch (Kiesel/Hexan:Ethylacetat = 9:1) gereinigt. Es werden 4,68 g 2-Brom-3-fluor-5-(4-octyloxyphenyl)pyrazin erhalten.

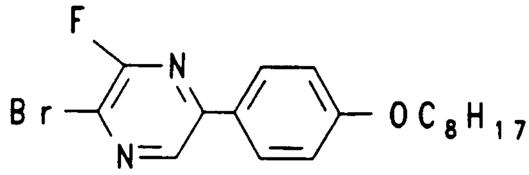

1d:

Zu 2,00 g (5,3 mmol) 2-Brom-3-fluor-5-(4-octyloxyphenyl)pyrazin und 0,03 g (0,06 mmol) [1,3-Bis(diphenylphosphino)propan]nickel[II]chlorid in 30 ml Tetrahydrofuran werden bei -10°C 7,95 mmol frisch bereitetes Octylmagnesiumbromid in 30 ml Tetrahydrofuran getropft und über Nacht gerührt, wobei sich das Reaktionsgemisch auf Raumtemperatur erwärmt. Nach Verteilen des Reaktionsgemisches zwischen wäßriger Ammoniumchloridlösung und Ether wird die organische Phase mit wäßriger Natriumchloridlösung gewaschen, über Natriumsulfat getrocknet, filtriert, vom Lösungsmittel befreit und chromatographisch (Kieselgel/Dichlormethan:Hexan = 7:3) gereinigt. Es werden 1,12 g 2-Fluor-3-octyl-6-(4-octyloxyphenyl)pyrazin erhalten.

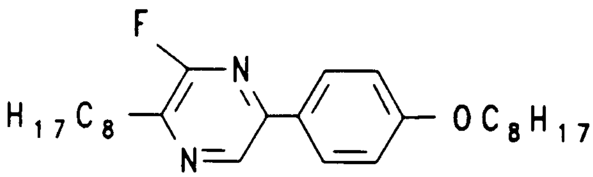

Die Verbindung zeigt folgende Phasenfolge:
X 42 $S_A$ 50 I

Beispiel 2:

2-Fluor-3,6-bis(4-octyloxyphenyl)pyrazin

Analog Beispiel 1b werden aus 2,00 g (5,25 mmol) 2-Brom-3-fluor-5-(4-octyloxyphenyl)pyrazin (hergestellt wie in Beispiel 1a - 1c beschrieben) und 1,31 g (5,25 mmol) 4-Octyloxyphenylboronsäure 1,96 g 2-Fluor-3,6-bis(4-octyloxyphenyl)pyrazin erhalten.

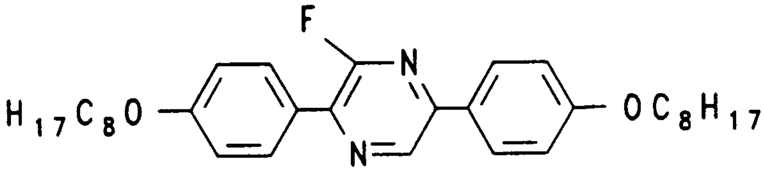

Die Verbindung zeigt folgende Phasenfolge:
X 89 $S_C$ 162 N 178 I

Beispiel 3:

2-Fluor-3-octyl-6-[4-(trans-4-pentylcyclohexyl)phenyl]pyrazin

3a:

Analog Beispiel 1b werden aus 2,17 g (16,41 mmol) 2-Chlor-6-fluorpyrazin (hergestellt wie in Beispiel 1a beschrieben) und 4,50 g (16,41 mmol) 4-(trans-4-Pentylcyclohexyl)phenylboronsäure 3,36 g 2-Fluor-6-[4-(trans-4-pentylcyclohexyl)phenyl]pyrazin erhalten.

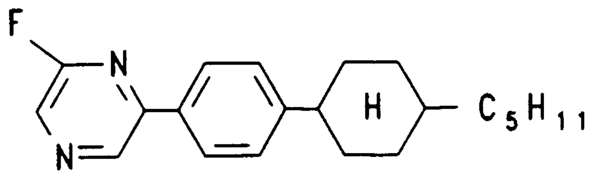

3b:

Analog Beispiel 1c werden aus 3,10 g (9,50 mmol) 2-Fluor-6-[4-(trans-4-pentylcyclohexyl)phenyl]pyrazin 2,75 g 2-Brom-3-fluor-5-[4-(trans-4-pentylcyclohexyl)phenyl]pyrazin erhalten.

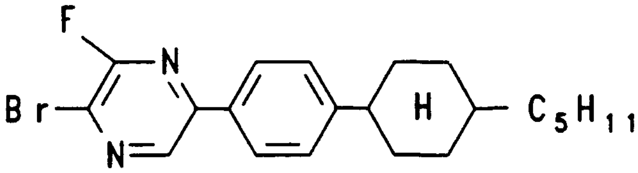

3c:

Analog Beispiel 1d werden aus 2,75 g (6,80 mmol) 2-Brom-3-fluor-5-[4-(trans-4-pentylcyclohexyl)phenyl]pyrazin und 10,2 mmol Octylmagnesiumbromid 1,53 g 2-Fluor-3-octyl-6-[4-(trans-4-pentylcyclohexyl)phenyl]pyrazin erhalten.

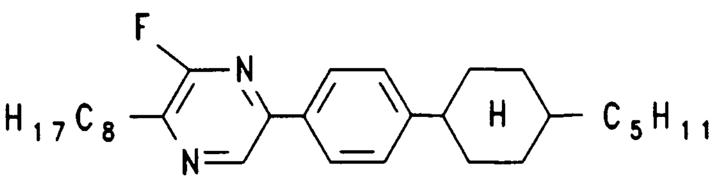

Die Verbindung zeigt folgende Phasenfolge:
X 48 $S_2$ 76 $S_A$ 128 I

Beispiel 4:

trans-4-Pentylcyclohexancarbonsäure-[4-(2-fluor-3-octylpyrazin-6-yl)phenyl]ester

4a:

Analog Beispiel 1b werden aus 8,86 g (66,85 mmol) 2-Chlor-6-fluorpyrazin (hergestellt wie in Beispiel 1a beschrieben) und 25,13 g (66,85 mmol) 4-tert-Butyldiphenylsilyloxyphenylboronsäure 19,90 g 2-Fluor-6-(4-tert-butyldiphenylsilyloxyphenyl)-pyrazin erhalten.

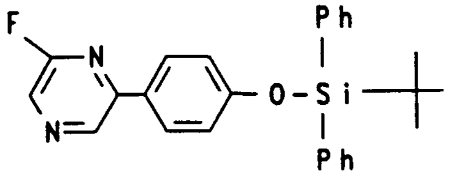

4b:

Analog Beispiel 1c werden aus 15,00 g (35,00 mmol) 2-Fluor-6-(4-tert-butyldiphenylsilyloxyphenyl)pyrazin 15,57 g 2-Brom-3-fluor-5-(4-tert-butyldiphenylsilyloxyphenyl)pyrazin erhalten.

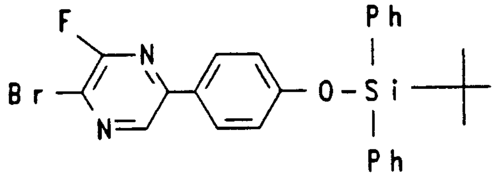

4c:

Analog Beispiel 1d werden aus 15,57 g (30,70 mmol) 2-Brom-3-fluor-5-(4-tert-butyldiphenylsilyloxyphenyl)pyrazin und 46,00 mmol Octylmagnesiumbromid 11,82 g 2-Fluor-3-octyl-6-(4-tert-butyldiphenylsilyloxyphenyl)pyrazin erhalten.

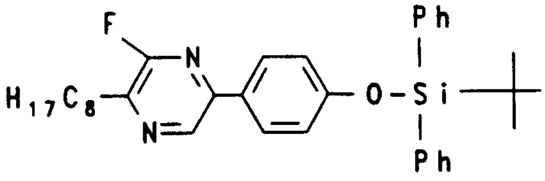

4d:

11,82 g (21,87 mmol) 2-Fluor-3-octyl-6-(4-tert-butyldiphenylsilyloxyphenyl)pyrazin und 43,74 ml einer 1 molaren Lösung von Tetra-n-butylammoniumfluorid in Tetrahydrofuran werden in 140 ml Tetrahydrofuran 1 h bei Raumtemperatur gerührt. Nach Verteilen des Reaktionsgemisches zwischen wäßriger Natriumchloridlösung und Ether wird die organische Phase mit wäßriger Natriumchloridlösung gewaschen, über Natriumsulfat getrocknet, vom Lösungsmittel befreit und aus n-Hexan umkristallisiert. Es werden 6,00 g 2-Fluor-3-octyl-6-(4-hydroxyphenyl)pyrazin erhalten.

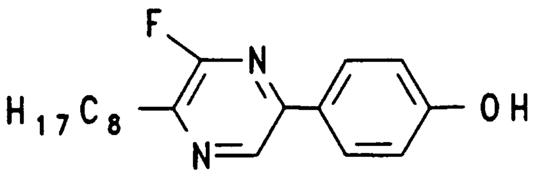

4e:

2,00 g (7,00 mmol) 2-Fluor-3-octyl-6-(4-hydroxyphenyl)pyrazin werden mit 1,44 g (7,00 mmol) Dicyclohexylcarbodiimid und wenig 4-Dimethylaminopyridin in 35 ml Dichlormethan 6 h bei Raumtemperatur gerührt. Nach der Abfiltration fester Bestandteile wird vom Lösungsmittel befreit und chromatographisch (Kieselgel/Hexan:Ethylacetat = 9:1) und durch Umkristallisation aus Acetonitril gereinigt. Es werden 1,94 g trans-4-Pentylcyclohexancarbonsäure-[4-(2-fluor-3-octylpyrazin-6-yl)phenyl]ester erhalten.

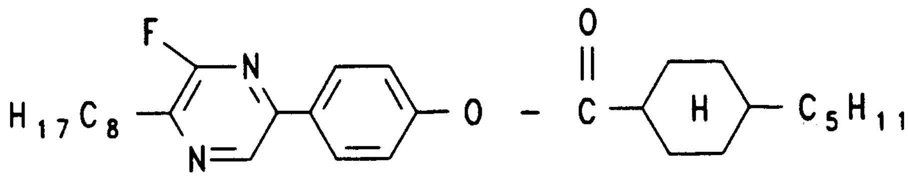

Die Verbindung zeigt folgende Phasenfolge:
X 58 $S_2$ 79 $S_A$ 157 I

Beispiel 5

2-Fluor-3-octyl-6-[4-(4-butyldimethylsilylbutyloxy)phenyl]pyrazin

5a:

Zu 3,04 g (11,60 mmol) Triphenylphosphin in 85 ml Tetrahydrofuran werden bei 0°C 1,82 ml (11,60 mmol) Diethylazodicarboxylat getropft und 0,5 h bei 0°C gerührt. Anschließend werden 2,00 g (7,00 mmol) 2-Fluor-3-octyl-6-(4-hydroxyphenyl)pyrazin (hergestellt wie in Beispiel 4a bis 4d beschrieben) und 2,46 g (11,60 mmol) 4-Butyldimethylsilylbutanol zugegeben und 3 h bei Raumtemperatur gerührt. Nach Einengen des Reaktionsgemisches wird chromatographisch (Kieselgel/Dichlormethan:Hexan = 8:2) gereinigt. Es werden 1,47 g 2-Fluor-3-octyl-6-[4-(4-butyldimethylsilylbutyloxy)phenyl]pyrazin erhalten.

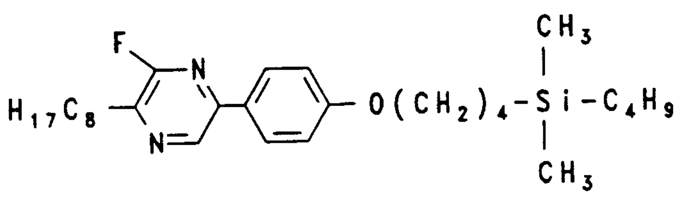

Der Brechungsindex $\eta_D$ der Verbindung beträgt bei 28°C 1.5212.

Beispiel 6

[(2S,3S)-3-Pentyloxiran-2-yl]methyl-[4-(2-fluor-3-octylpyrazin-6-yl)phenyl]ether

6a:

Analog Beispiel 5a werden aus 3,04 g (11,60 mmol) Triphenylphosphin, 1,82 ml (11,60 mmol) Diethylazodicarboxylat, 2,00 g (7,00 mmol) 2-Fluor-3-octyl-6-(4-hydroxyphenyl)pyrazin und 1,67 g (11,60 mmol) (2S,3S)-3-Pentyloxiran-2-ylmethanol 1,20 g [(2S,3S)-3-Pentyloxiran-2-yl]methyl-[4-(2-fluor-3-octylpyrazin-6 yl)phenyl]ether mit $[\alpha]^{20}_{D}$ (c = 0,995 in $CHCl_3$) = -16,58 erhalten.

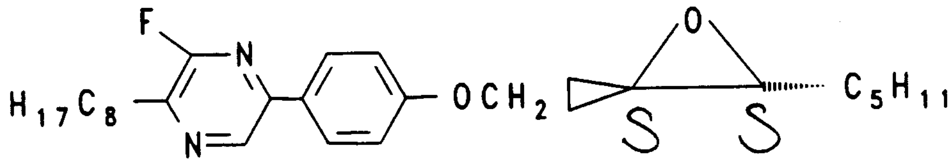

Die Verbindung zeigt folgende Phasenfolge:
X 64 $S_A$ 80 I

Anwendungsbeispiel 1

a) Eine ferroelektrische Mischung, die aus den Komponenten

| | |
|---|---|
| 5-Octyloxy-2-(4-hexyloxyphenyl)pyrimidin | 22,8 Mol-% |
| 5-Octyloxy-2-(4-butyloxyphenyl)pyrimidin | 24,0 Mol-% |
| 5-Octyloxy-2-(4-decyloxyphenyl)pyrimidin | 19,2 Mol-% |
| 5-Octyloxy-2-(4-octyloxyphenyl)pyrimidin | 10,5 Mol-% |
| trans-4-Pentyl-cyclohexancarbonsäure-(4-(5-decylpyrimidin-2-yl))phenylester | 13,5 Mol-% |
| [(2S,3S)-3-Pentyloxiran-2-yl]methyl-[4-(2-fluor-3-octylpyrazin-6-yl)phenyl]ether | 10,0 Mol-% |

besteht, zeigt folgende flüssigkristalline Phasenbereiche:

$S_C$* 80 $S_A$ 94 N* 100 I

Sie weist bei einer Temperatur von 20°C eine spontane Polarisation von 12,4 $nC/cm^2$ auf und schaltet bei einer Feldstärke von 10 V/$\mu$m mit einer Schaltzeit von 212 $\mu$s. Die Mischung zeigt in der nematischen Phase eine helikale Verdrillungskraft (HTP) zwischen -0.45 $\mu m^{-1}$ und -0.39 $\mu m^{-1}$.

b) Im Vergleich dazu weist eine bekannte flüssigkristalline Mischung (DE 38 31 226.3), die sich von der obengenannten Mischung nur dadurch unterscheidet, daß sie keinen Dotierstoff enthält, folgende Phasenbereiche auf:

$S_C$* 84 $S_A$ 93 N* 105 I

Die Mischung belegt, daß mit den erfindungsgemäßen Verbindungen schnell schaltende ferroelektrische Mischungen herstellbar sind.

Anwendungsbeispiel 2

a) eine Mischung, die aus den Komponenten

| | |
|---|---|
| 5-Heptyl-2-(4-pentyloxyphenyl)pyrimidin | 9,48 Mol-% |
| 5-Heptyl-2-(4-heptyloxyphenyl)pyrimidin | 14,23 Mol-% |
| 5-Heptyl-2-(4-butyloxyphenyl)pyrimidin | 15,16 Mol-% |
| 5-(6-cyclopropyl)hexyloxy-2-(4-octyloxyphenyl)pyrimidin | 14,38 Mol-% |
| trans-4-Pentylcyclohexylmethyloxy-[5-(8-cyclopropyloctyloxy)pyrimidin-2-yl-phenyl]ether | 6,58 Mol-% |
| trans-4-Pentylcyclohexancarbonsäure-[5-(8-cyclopropyloctyl)pyrimidin-2-yl-phenyl]ester | 11.40 Mol-% |
| 5-Octyl-2-[4-(6-cyclopropyl)hexylcarbonyloxy-phenyl]-pyrimidin | 21,28 Mol-% |
| 2-Fluor-3-octyl-6-[4-(trans-4-pentylcyclohexyl)phenyl]pyrazin | 7,5 Mol-% |

besteht, zeigt folgende flüssigkristalline Phasenbereiche:

$S_C$ 55 $S_A$ 74 N 94 I

b) Im Vergleich dazu weist die flüssigkristalline Mischung, die sich von der obengenannten Mischung nur dadurch unterscheidet, daß sie keine erfindungsgemäße Verbindung aufweist, folgende Phasenbereiche auf:

$S_C$ 61 N 91 I

Der Vergleich zeigt, daß schon bei geringen Zusatzmengen der erfindungsgemäßen Verbindung in flüssigkristallinen Mischungen eine $S_A$ Phase erzeugt werden kann.

**Patentansprüche**

1. 2-Fluorpyrazin der allgemeinen Formel (I)

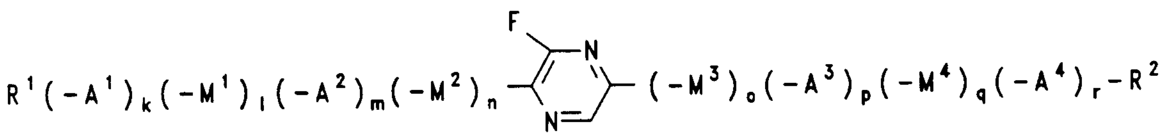

(I)

bei dem die Symbole folgende Bedeutung haben:

$R^1$, $R^2$ unabhängig voneinander H, F, Cl, CN, NCS, $-CF_3$, $-OCF_3$, $-OCHF_2$ oder geradkettiges oder verzweigtes Alkyl mit 1 bis 16 C-Atomen (mit oder ohne asymmetrischem C-Atom), wobei auch eine oder zwei nicht-benachbarte $-CH_2$-Gruppen jeweils durch -O-, -S-, -CO-, -CO-O-, -O-CO-, -CO-S-, -S-CO-, -O-CO-O, -CH=CH-, -C≡C-,

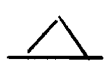

oder $-Si(CH_3)_2$- ersetzt sein können, und wobei auch ein oder mehrere H-Atome des Alkylrestes jeweils durch F, Cl, Br oder CN substituiert sein können, oder eine der nachfolgenden chiralen Gruppen:

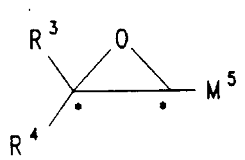, 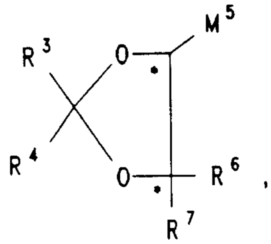, 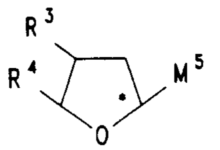, 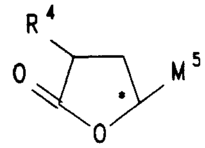,

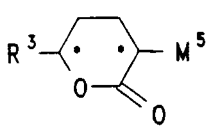, 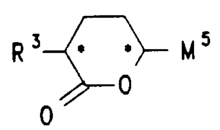, 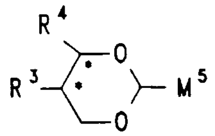, 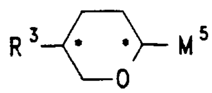,

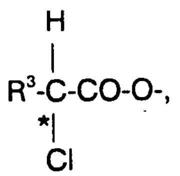, 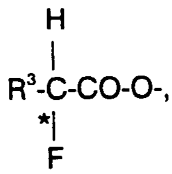, 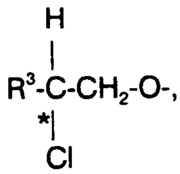, 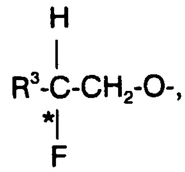,

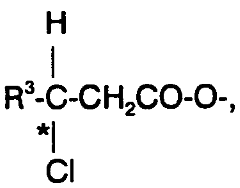, 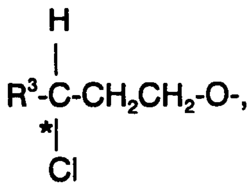,

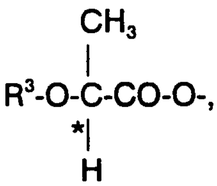, 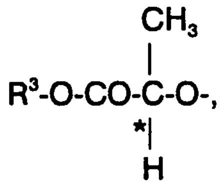,

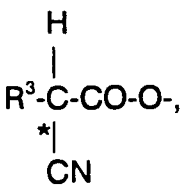, 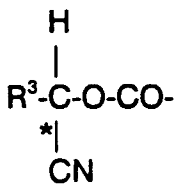

$A^1$, $A^2$, $A^3$, $A^4$ gleich oder verschieden 1,4-Phenylen, Pyrazin-2,5-diyl, Pyridazin-3,6-diyl, Pyridin-2,5-diyl, Pyrimidin-2,5-diyl, wobei ein oder zwei H-Atome jeweils durch F, Cl und/oder CN ersetzt sein können, trans-1,4-Cyclohexylen, bei dem ein oder zwei H-Atome durch CN und/oder $CH_3$ ersetzt sein können, (1,3,4)-Thiadiazol-2,5-diyl, 1,3-Dioxan-2,5-diyl, Naphthalin-2,6-diyl, Bicyclo[2.2.2.]octan-1,4-diyl oder 1,3-Dioxaborinan-2,5-diyl;

$M^1$, $M^2$, $M^3$, $M^4$ gleich oder verschieden -O-, -S-, -CO-, -CO-O-, -O-CO-, -CO-S-, -S-CO-, -O-CO-O-, $-CH_2-O-$, $-O-CH_2-$, $-CH_2-CH_2-$, -CH=CH- oder -C≡C-;

$R^3$, $R^4$, $R^6$, $R^7$ unabhängig voneinander H oder geradkettiges oder verzweigtes Alkyl mit 1 bis 16 C-Atomen;

oder $R^3$ und $R^4$ zusammen auch $-(CH_2)_4-$ oder $-(CH_2)_5-$, wenn als Substituenten an ein Dioxolan-System gebunden;

$M^5$ $-CH_2-O-$, -CO-O-, $-O-CH_2-$, -O-CO- oder eine Einfachbindung; k, l, m, n, o, p, q, r Null oder 1, unter der Bedingung, daß die Summe k+m+p+r kleiner 4 und größer Null ist.

**2.** 2-Fluorpyrazin gemäß Anspruch 1, dadurch gekennzeichnet, daß die Symbole folgende Bedeutung haben:

$R^1$, $R^2$ unabhängig voneinander H, F, CN, oder geradkettiges oder verzweigtes Alkyl mit 1 bis 16 C-Atomen (mit oder ohne asymmetrischem C-Atom), wobei auch eine $-CH_2$-Gruppe durch -O-, -S-, -CO-, -CO-O-, -O-CO-, -O-CO-O-, -CH=CH-, -C≡C-,

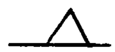

oder $-Si(CH_3)_2-$ ersetzt sein kann, oder eine der nachfolgenden chiralen Gruppen:

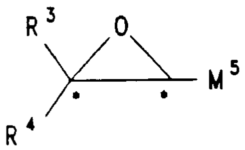 , 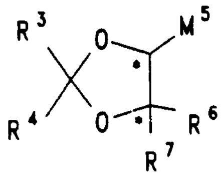 , 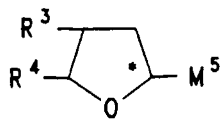 , 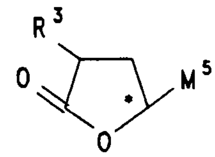 ,

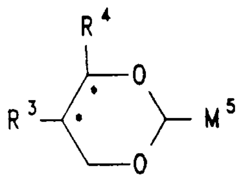 ,

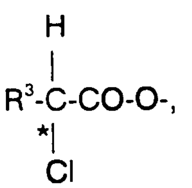 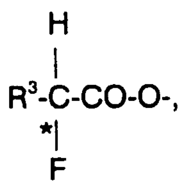 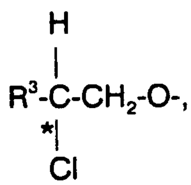 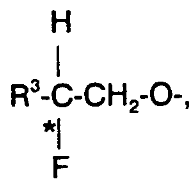

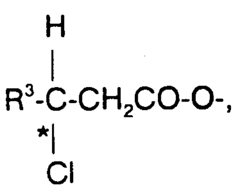 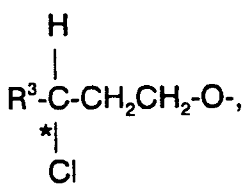

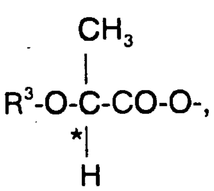 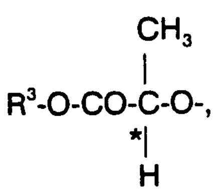

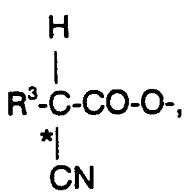 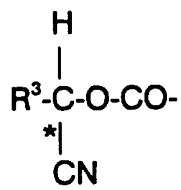

$A^1$, $A^2$, $A^3$, $A^4$ gleich oder verschieden 1,4-Phenylen, Pyrazin-2,5-diyl, Pyridazin-3,6-diyl, Pyridin-2,5-diyl, Pyrimidin-2,5-diyl, wobei ein oder zwei H-Atome jeweils durch F ersetzt sein können, trans-1,4-Cyclohexylen, (1,3,4)-Thiadiazol-2,5-diyl oder Naphthalin-2,6-diyl, 1,3-Dioxan-2,5-diyl oder Bicyclo[2.2.2.]octan-1,4-diyl;
$M^1$, $M^2$, $M^3$, $M^4$ gleich oder verschieden -O-, -CO-, -CO-O-, -O-CO-, $-CH_2-O-$, $-O-CH_2-$, $-CH_2-CH_2-$, -CH=CH- oder -C≡C-;
$R^3$, $R^4$, $R^6$, $R^7$ unabhängig voneinander H oder geradkettiges oder verzweigtes Alkyl mit 1 bis 16 C-Atomen;
$R^3$ und $R^4$ zusammen auch $-(CH_2)_4-$ oder $-(CH_2)_5-$ wenn als Substituenten an einem Dioxolan-System gebunden;
$M^5$ $-CH_2-O-$, -CO-O-, $-O-CH_2-$, -O-CO- oder eine Einfachbindung.

**3.** 2-Fluorpyrazin gemäß Anspruch 1, dadurch gekennzeichnet, daß die Symbole folgende Bedeutung haben:

$R^1$, $R^2$ unabhängig voneinander H, F, CN oder geradkettiges oder verzweigtes Alkyl mit 1 bis 16 C-Atomen (mit oder ohne asymmetrischem C-Atom), wobei auch eine $-CH_2$-Gruppe durch -O-, -CO-, -CO-O-, -O-CO-, -CH=CH-,

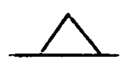

oder $-Si(CH_3)_2-$ ersetzt sein können, oder eine der nachfolgenden chiralen Gruppen:

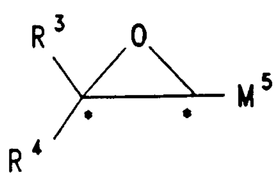 , 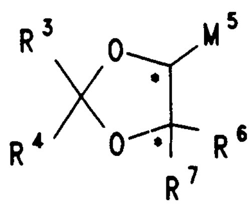 , 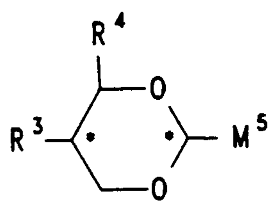 ,

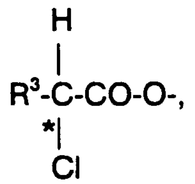 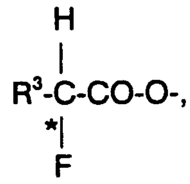

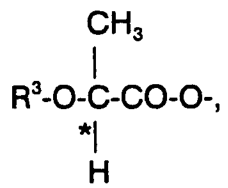 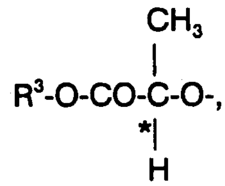

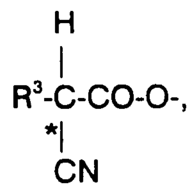 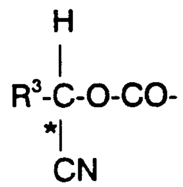

$A^1$, $A^2$, $A^3$, $A^4$ gleich oder verschieden 1,4-Phenylen, Pyrazin-2,5-diyl, Pyridazin-3,6-diyl, Pyridin-2,5-diyl, Pyrimidin-2,5-diyl, bei dem ein H-Atom durch F ersetzt sein kann, trans-1,4-Cyclohexylen, Naphthalin-2,6-diyl oder 1,3-Dioxan-2,5-diyl;

$M^1$, $M^2$, $M^3$, $M^4$ gleich oder verschieden -O-, -CO-O-, -O-CO-, -O-$CH_2$-, -$CH_2$-O-, -$CH_2$-$CH_2$- oder -CH=CH-;

$R^3$, $R^4$, $R^6$, $R^7$ unabhängig voneinander H oder geradkettiges oder verzweigtes Alkyl mit 1 bis 16 C-Atomen. $R^3$ und $R^4$ zusammen auch -$(CH_2)_4$- oder -$(CH_2)_5$- wenn als Substituenten an einem Dioxolan-System gebunden;

$M^5$ -$CH_2$-O-, -CO-O-, -O-$CH_2$-, -O-CO- oder eine Einfachbindung.

4. 2-Fluorpyrazin gemäß Anspruch 1, dadurch gekennzeichnet, daß die Symbole in Formel (I) folgende Bedeutung haben:

$R^1$, $R^2$ unabhängig voneinander H oder Alkyl mit 1 bis 16 C-Atomen, wobei eine -$CH_2$-Gruppe durch -O- ersetzt sein kann, oder die chirale Gruppe

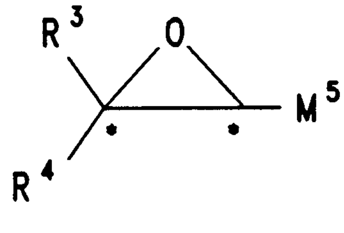

$A^1$, $A^2$, $A^3$, $A^4$ gleich oder verschieden 1,4-Phenylen, Pyrimidin-2,5-diyl, 1,4-Cyclohexylen,

$M^1$, $M^2$, $M^3$, $M^4$ gleich oder verschieden -O-, -COO-, -$CH_2$-O-, -O-$CH_2$-,

$M^5$ gleich -$CH_2$-O- oder -COO- und

$R^3$, $R^4$ unabhängig voneinander H oder geradkettiges Alkyl mit 1 bis 10 C-Atomen.

5. Verwendung eines Fluorpyrazins gemäß Anspruch 1 als Komponente in Flüssigkristallmischungen.

6. Flüssigkristalline Mischung bestehend aus mindestens zwei Komponenten, dadurch gekennzeichnet, daß sie als eine Komponente ein Fluorpyrazin gemäß Anspruch 1 enthält.

7. Ferroelektrische Flüssigkristallmischung bestehend aus 2 bis 20 Komponenten, dadurch gekennzeichnet, daß sie als eine Komponente eine Verbindung gemäß Anspruch 1 enthält.

8. Nematische Flüssigkristallmischung bestehend aus 2 bis 20 Komponenten, dadurch gekennzeichnet, daß sie als eine Komponente eine Verbindung gemäß Anspruch 1 enthält.

9. Ferroelektrische Flüssigkristallmischung bestehend aus 2 bis 20 Komponenten, dadurch gekennzeichnet, daß sie als eine Komponente eine Verbindung gemäß Anspruch 2 enthält.

10. Ferroelektrische Flüssigkristallmischung bestehend aus 2 bis 20 Komponenten, dadurch gekennzeichnet, daß sie als eine Komponente eine Verbindung gemäß Anspruch 3 enthält.

11. Ferroelektrische Flüssigkristallmischung bestehend aus 2 bis 20 Komponenten, dadurch gekennzeichnet, daß sie als eine Komponente eine Verbindung gemäß Anspruch 4 enthält.

12. Ferroelektrische Flüssigkristallmischung bestehend aus 2 bis 20 Komponenten, dadurch gekennzeichnet, daß sie 1 bis 25 Mol-% mindestens einer Verbindung gemäß Formel (I) aus Anspruch 1 enthält.

13. Ferroelektrische Schalt- und Anzeigevorrichtung enthaltend Trägerplatten, Elektroden, mindestens einen Polarisator, mindestens eine Orientierungsschicht sowie ein flüssigkristallines Medium, dadurch gekennzeichnet, daß das flüssigkristalline Medium eine Flüssigkristallmischung gemäß Anspruch 6 ist.

14. Verfahren zur Herstellung der Verbindung der Formel (I) nach Anspruch 1, dadurch gekennzeichnet, daß in mehrstufigen Umsetzungen die Seitenketten $R^1(-A^1)_k(-M^1)_l(-A^2)_m(-M^2)_n$- und $-(M^3)_o-(A^3)_p-(M^4)_q-(A^4)_r-R^2$ in 3- bzw. 6-Position des Pyrazinringes von 2,6-Dichlorpyrazin eingebracht werden.

15. Verfahren nach Anspruch 14, dadurch gekennzeichnet, daß 2,6-Dichlorpyrazin (II) mit einem Fluorid-Reagenz unter Verwendung katalytischer Mengen eines Komplexbildners zu 2,6-Difluorpyrazin (III) umgesetzt wird, 2,6-Difluorpyrazin (III) mit einer Metallverbindung von $(-M^3)_o(-A^3)_p(-M^4)_q(-A^4)_r-R^2$ zur Verbindung der Formel (V) reagiert und anschließend die Verbindung der Formel (V) zum 2-Fluorpyrazinderivat der Formel I nach Anspruch 1 umgesetzt wird.

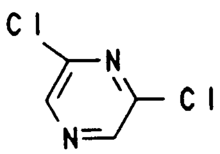 (II) 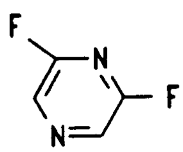 (III)

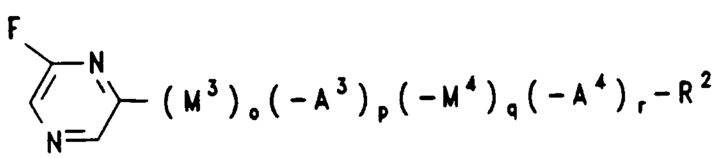 (V)

16. Verfahren nach Anspruch 14, dadurch gekennzeichnet, daß 2,6-Dichlorpyrazin (II) mit einem Fluorid-Reagenz unter vermindertem Druck in Gegenwart eines Komplexbildners zu 2-Chlor-6-fluorpyrazin (IV) umgesetzt wird, 2-Chlor-6-fluorpyrazin (IV) mit einem metallorganischen Derivat von $(-M^3)_o(-A^3)_p(-M^4)_q(-A^4)_r-R^2$ unter Verwendung von Übergangsmetallkatalysatoren zur Verbindung der Formel (V) umgesetzt und weiter zum 2-Fluorpyrazinderivat der Formel I nach Anspruch 1 umgesetzt wird.

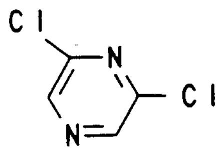 (II) 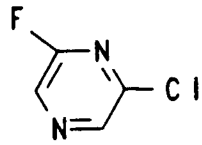 (IV)

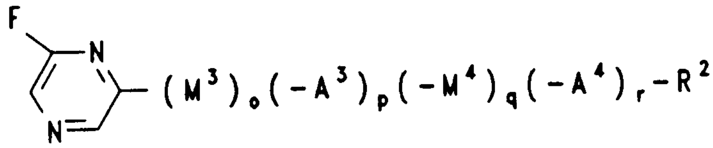 (V)

17. Verfahren nach Anspruch 14, dadurch gekennzeichnet, daß eine Verbindung der Formel (V) durch Behandlung mit einer Lithiumverbindung zur Verbindung der Formel VI umgesetzt wird,

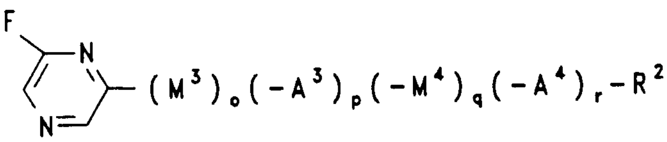 (V) 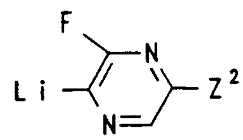

(VI)

und das Reaktionsprodukt durch ein- oder mehrstufige Umsetzung zum 2-Fluorpyrazinderivat der Formel (I) nach Anspruch 1 umgesetzt wird.

## Claims

1. A 2-fluoropyrazine of the formula (I)

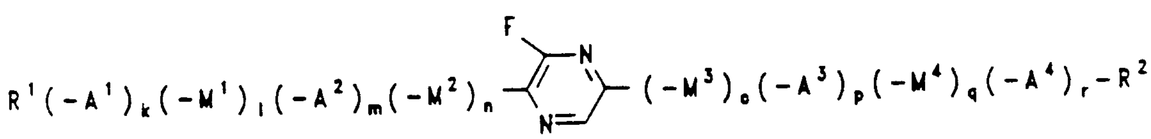

(I)

in which the symbols have the following meanings:

$R^1$ and $R^2$, independently of one another, are H, F, Cl, CN, NCS, $-CF_3$, $-OCF_3$, $-OCHF_2$ or straight-chain or branched alkyl having 1 to 16 carbon atoms (with or without an asymmetrical carbon atom), it also being possible for one or two non-adjacent $-CH_2$-groups each to be replaced by -O-, -S-, -CO-, -CO-O-, -O-CO-, -CO-S-, -S-CO-, -O-CO-O-, -CH=CH-, -C≡C-,

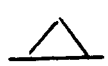

or $-Si(CH_3)_2-$, and it also being possible for one or more hydrogen atoms of the alkyl radical each to be substituted by F, Cl, Br or CN, or one of the following chiral groups:

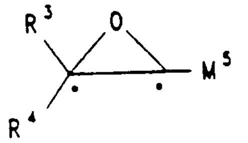, 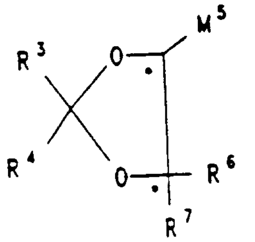, 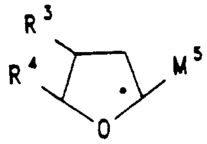, 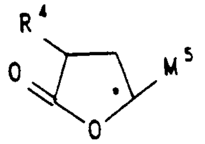,

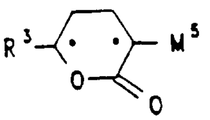, 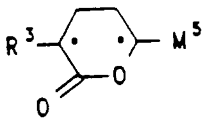, 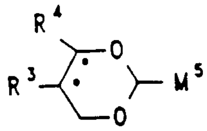, 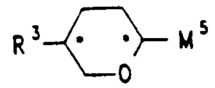,

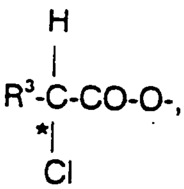, 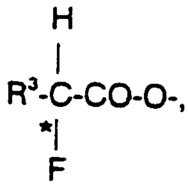, 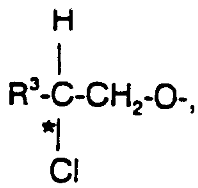, 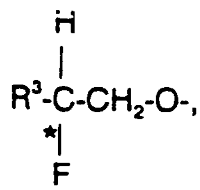,

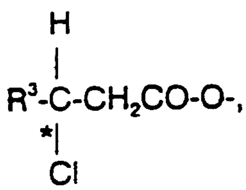, 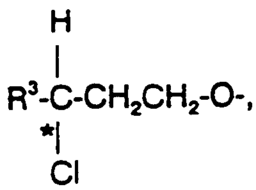,

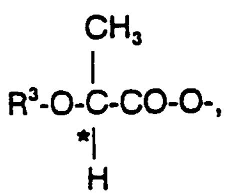, 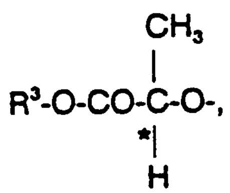,

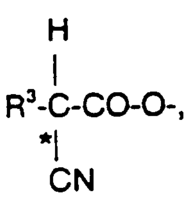, 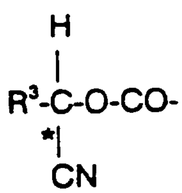

$A^1$, $A^2$, $A^3$ and $A^4$ are identical or different 1,4-phenylene, pyrazine-2,5-diyl, pyridazine-3,6-diyl, pyridine-2,5-diyl or pyrimidine-2,5-diyl, it being possible for one or two hydrogen atoms each to be replaced by F, Cl and/or CN, or are trans-1,4-cyclohexylene, in which one or two hydrogen atoms may be replaced by CN and/or $CH_3$, or are 1,3,4-thiadiazole-2,5-diyl, 1,3-dioxane-2,5-diyl, naphthalene-2,6-diyl, bicyclo[2.2.2]octane-1,4-diyl or 1,3-dioxaborinane-2,5-diyl;

$M^1$, $M^2$, $M^3$ and $M^4$ are identical or different -O-, -S-, -CO-, -CO-O-, -O-CO-, -CO-S-, -S-CO-, -O-CO-O-, $-CH_2-O-$, $-O-CH_2-$, $-CH_2-CH_2-$, -CH=CH- or -C≡C-;

$R^3$, $R^4$, $R^6$ and $R^7$, independently of one another, are H or straight-chain or branched alkyl having 1 to 16 carbon atoms;

or $R^3$ and $R^4$ together are alternatively $-(CH_2)_4-$ or $-(CH_2)_5-$ if bonded as substituents to a dioxolane system;

$M^5$ is $-CH_2-O-$, -CO-O-, $-O-CH_2-$, -O-CO- or a single bond; and

k, l, m, n, o, p, q and r are zero or 1, with the condition that the sum of k+m+p+r is less than 4 and greater than zero.

**2.** A 2-fluoropyrazine as claimed in claim 1, wherein the symbols have the following meanings:

$R^1$ and $R^2$, independently of one another, are H, F, CN or straight-chain or branched alkyl having 1 to 16 carbon atoms (with or without an asymmetrical carbon atom), it also being possible for a $-CH_2-$group to be replaced by -O-, -S-, -CO-, -CO-O-, -O-CO-, -O-CO-O-, -CH=CH-, -C≡C-,

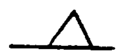

or $-Si(CH_3)_2-$, or one of the following chiral groups:

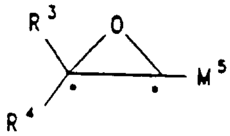 , 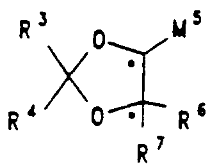 , 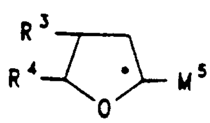 , 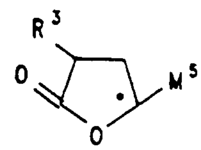 ,

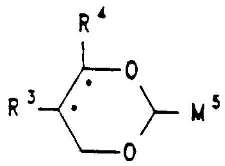 ,

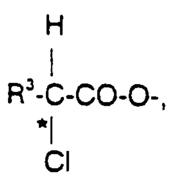 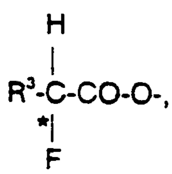 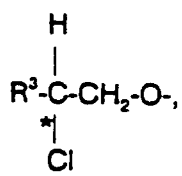 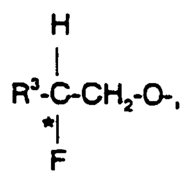

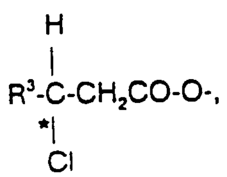

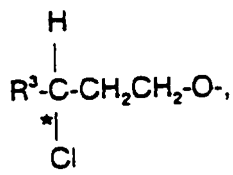

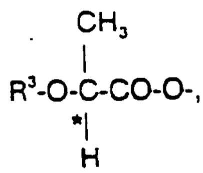

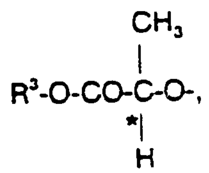

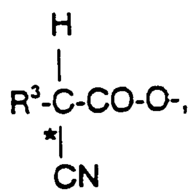

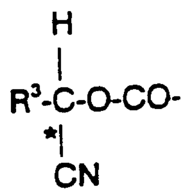

$A^1$, $A^2$, $A^3$ and $A^4$ are identical or different 1,4-phenylene, pyrazine-2,5-diyl, pyridazine-3,6-diyl, pyridine-2,5-diyl or pyrimidine-2,5-diyl, it being possible for one or two hydrogen atoms each to be replaced by F, or are trans-1,4-cyclohexylene, 1,3,4-thiadiazole-2,5-diyl, naphthalene-2,6-diyl, 1,3-dioxane-2,5-diyl or bicyclo[2.2.2]octane-1,4-diyl;

$M^1$, $M^2$, $M^3$ and $M^4$ are identical or different -O-, -CO-, -CO-O-, -O-CO-, $-CH_2-O-$, $-O-CH_2-$, $-CH_2-CH_2-$, -CH=CH- or -C≡C-;

$R^3$, $R^4$, $R^6$ and $R^7$, independently of one another, are H or straight-chain or branched alkyl having 1 to 16 carbon atoms; $R^3$ and $R^4$ together are alternatively $-(CH_2)_4-$ or $-(CH_2)_5-$ if bonded as substituents to a dioxolane system; and

$M^5$ is $-CH_2-O-$, -CO-O-, $-O-CH_2-$, -O-CO- or a single bond.

**3.** A 2-fluoropyrazine as claimed in claim 1, wherein the symbols have the following meanings:

$R^1$ and $R^2$, independently of one another, are H, F, CN or straight-chain or branched alkyl having 1 to 16 carbon atoms (with or without an asymmetrical carbon atom), it also being possible for a $-CH_2-$group to be replaced by -O-, -CO-, -CO-O-, -O-CO-, -CH=CH-,

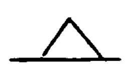

or $-Si(CH_3)_2-$, or one of the following chiral groups:

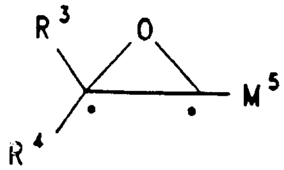 , 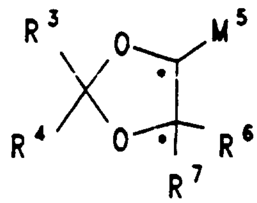 , 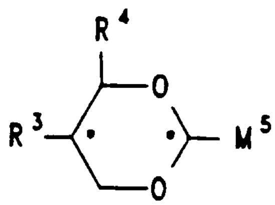 ,

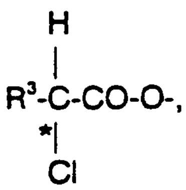 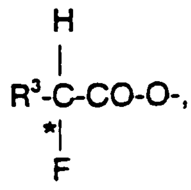

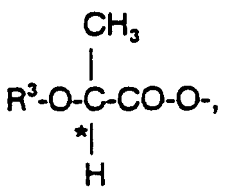 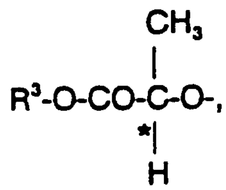

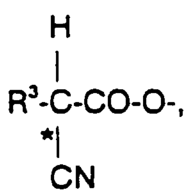 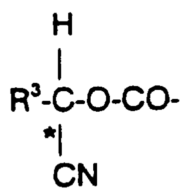

$A^1$, $A^2$, $A^3$ and $A^4$ are identical or different 1,4-phenylene, pyrazine-2,5-diyl, pyridazine-3,6-diyl, pyridine-2,5-diyl or pyrimidine-2,5-diyl, in which a hydrogen atom may be replaced by F, or are trans-1,4-cyclohexylene, naphthalene-2,6-diyl or 1,3-dioxane-2,5-diyl;

$M^1$, $M^2$, $M^3$ and $M^4$ are identical or different -O-, -CO-O-, -O-CO-, -O-$CH_2$-, -$CH_2$-O-, -$CH_2$-$CH_2$- or -CH=CH-;

$R^3$, $R^4$, $R^6$ and $R^7$, independently of one another, are H or straight-chain or branched alkyl having 1 to 16 carbon atoms; $R^3$ and $R^4$ together are alternatively -$(CH_2)_4$- or -$(CH_2)_5$- if bonded as substituents to a dioxolane system; and

$M^5$ is -$CH_2$-O-, -CO-O-, -O-$CH_2$-, -O-CO- or a single bond.

**4.** A 2-fluoropyrazine as claimed in claim 1, wherein the symbols in the formula (I) have the following meanings:

$R^1$ and $R^2$, independently of one another, are H or alkyl having 1 to 16 carbon atoms, it being possible for a -$CH_2$- group to be replaced by -O-, or the chiral group

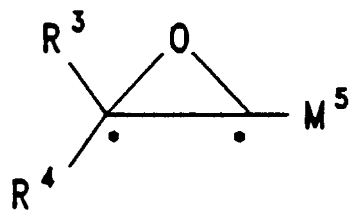

$A^1$, $A^2$, $A^3$ and $A^4$ are identical or different 1,4-phenylene, pyrimidine-2,5-diyl or 1,4-cyclohexylene,

$M^1$, $M^2$, $M^3$ and $M^4$ are identical or different -O-, -COO-, $-CH_2-O-$ or $-O-CH_2-$,

$M^5$ is $-CH_2O-$ or -COO-, and

$R^3$ and $R^4$, independently of one another, are H or straight-chain alkyl having 1 to 10 carbon atoms.

**5.** The use of a fluoropyrazine as claimed in claim 1 as a component in liquid-crystal mixtures.

**6.** A liquid-crystalline mixture comprising at least two components, wherein one component is a fluoropyrazine as claimed in claim 1.

**7.** A ferroelectric liquid-crystal mixture comprising 2 to 20 components, wherein one component is a compound as claimed in claim 1.

**8.** A nematic liquid-crystal mixture comprising 2 to 20 components, wherein one component is a compound as claimed in claim 1.

**9.** A ferroelectric liquid-crystal mixture comprising 2 to 20 components, wherein one component is a compound as claimed in claim 2.

**10.** A ferroelectric liquid-crystal mixture comprising 2 to 20 components, wherein one component is a compound as claimed in claim 3.

**11.** A ferroelectric liquid-crystal mixture comprising 2 to 20 components, wherein one component is a compound as claimed in claim 4.

**12.** A ferroelectric liquid-crystal mixture comprising 2 to 20 components, which contains from 1 to 25 mol% of at least one compound as claimed in formula (I) from claim 1.

**13.** A ferroelectric switching and display device containing outer plates, electrodes, at least one polarizer, at least one alignment layer and a liquid-crystalline medium, wherein the liquid-crystalline medium is a liquid-crystal mixture as claimed in claim 6.

**14.** A process for the preparation of the compound of the formula (I) as claimed in claim 1, wherein the side chains $R^1(-A^1)_k(-M^1)_l(-A^2)_m(-M^2)_n-$ and $-(M^3)_o-(A^3)_p-(M^4)_q-(A^4)_r-R^2$ are introduced into the 3- or 6-position of the pyrazine ring of 2,6-dichloropyrazine in a multistep reaction.

**15.** The process as claimed in claim 14, wherein 2,6-dichloropyrazine (II) is reacted with a fluoride reagent using catalytic amounts of a complexing agent to give 2,6-difluoropyrazine (III), 2,6-difluoropyrazine (III) is reacted with a metal compound of $(-M^3)_o(-A^3)_p(-M^4)_q(-A^4)_r-R^2$ to give the compound of the formula (V), and the compound of the formula (V) is subsequently converted to the 2-fluoropyrazine derivative of the formula I as claimed in claim 1.

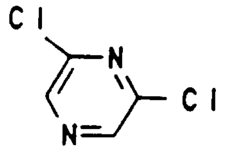 (II) 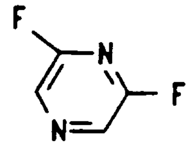 (III)

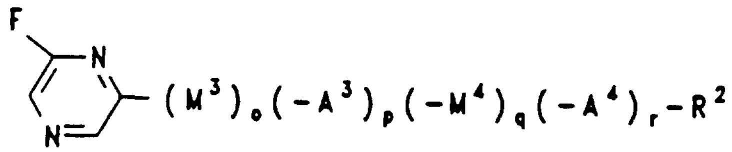 (V)

16. The process as claimed in claim 14, wherein 2,6-dichloropyrazine (II) is reacted with a fluoride reagent under reduced pressure in the presence of a complexing agent to give 2-chloro-6-fluoropyrazine (IV), 2-chloro-6-fluoropyrazine (IV) is reacted with an organometallic derivative of $(-M^3)_o(-A^3)_p(-M^4)_q(-A^4)_r-R^2$ using transition-metal catalysts to give the compound of the formula (V), and this is further converted to the 2-fluoropyrazine derivative of the formula I as claimed in claim 1.

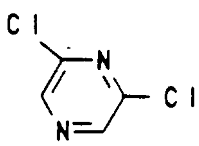 (II) 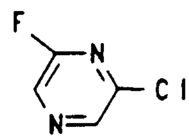 (IV)

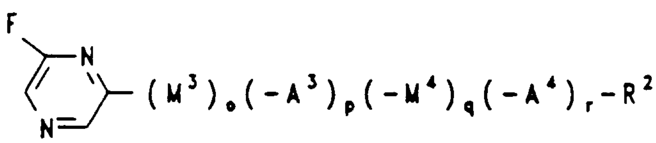 (V)

17. The process as claimed in claim 14, wherein a compound of the formula (V) is converted to the compound of the formula (VI) by treatment with a lithium compound,

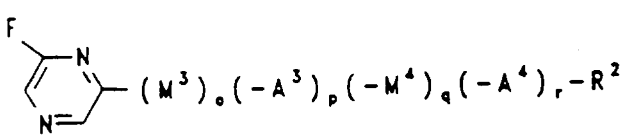 (V) 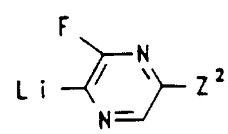

(VI)

and the reaction product is converted to the 2-fluoropyrazine derivative of the formula (I) as claimed in claim 1 by a single- or multistep reaction.

## Revendications

1. 2-Fluoropyrazine de formule I

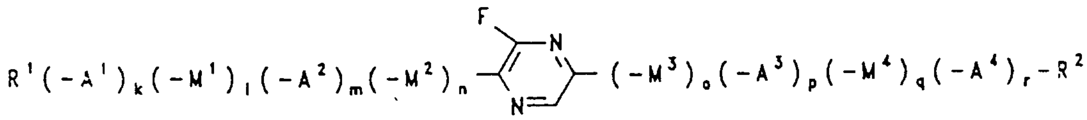

(I)

dans laquelle les symboles ont les significations suivantes :

$R^1$, $R^2$, indépendamment l'un de l'autre, représentent H, F, Cl, CN, NCS, $-CF_3$, $-OCF_3$, $-OCHF_2$ ou alkyle linéaire ou ramifié avec 1 à 16 atomes de carbone (avec ou sans atome de carbone asymétrique), un ou deux groupes $-CH_2$-voisins pouvant aussi être remplacés chacun par -O-, -S-, -CO-, -CO-O-, -O-CO-, -CO-S-, -S-CO-, -O-CO-O-, -CH=CH-, -C≡C-,

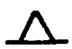

ou $-Si(CH_3)_2-$, un ou plusieurs atomes de H du radical alkyle pouvant aussi être rempacés chacun par F, Cl, Br ou CN, ou un des groupes chiraux suivants :

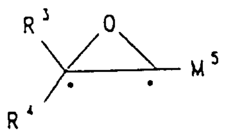,
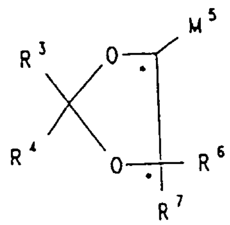,
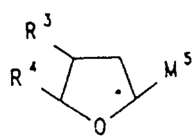,
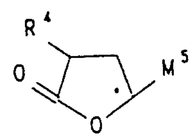,

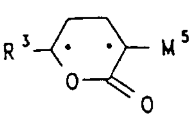,
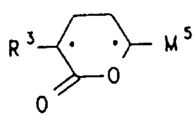,
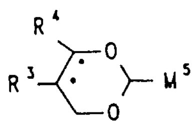,
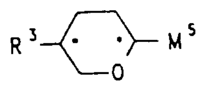,

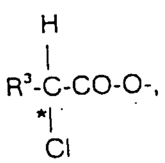
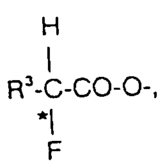
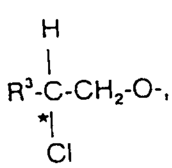
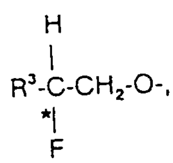

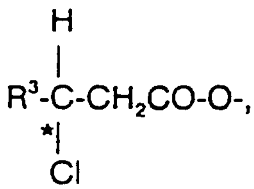

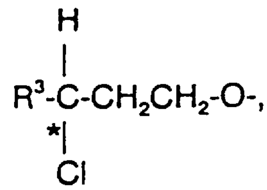

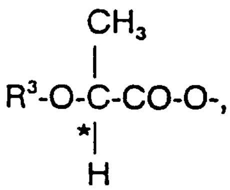

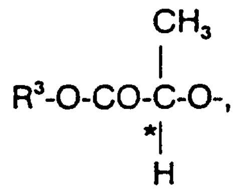

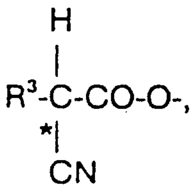

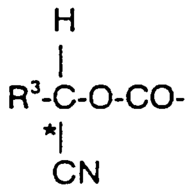

$A^1$, $A^2$, $A^3$, $A^4$, identiques ou différents, représentent 1,4-phénylène, pyrazine-2,5-diyle, pyridazine-3,6-diyle, pyridine-2,5-diyle, pyrimidine-2,5-diyle, un ou deux atomes de H pouvant être remplacés chacun par F, Cl et/ou CN, représente aussi trans-1,4-cyclohexylène, dans lequel un ou deux atomes H peuvent être remplacés par CN et/ou $CH_3$, (1,3,4)-thiadiazol-2,5-diyle, 1,3-dioxane-2,5-diyle, naphtalène-2,6-diyle, bicyclo[2.2.2.]octane-1,4-diyle ou 1,3-dioxaborinane-2,5-diyle ;

$M^1$, $M^2$, $M^3$, $M^4$, identiques ou différents, représentent -O-, -S-, -CO-, -CO-O-, -O-CO-, -CO-S-, -S-CO-, -O-CO-O-, $-CH_2-O-$, $-O-CH_2-$, $-CH_2-CH_2-$, -CH=CH- ou -C≡C- ;

$R^3$, $R^4$, $R^6$, $R^7$, indépendamment les uns des autres, représentent H ou alkyle linéaire ou ramifié avec 1 à 16 atomes de carbone ;

ou $R^3$ et $R^4$ ensemble représentent aussi $-(CH_2)_4-$ ou $-(CH_2)_5-$ lorsqu'ils sont liés comme substituants à un système dioxolane ;

$M^5$ représente $-CH_2-O-$, -CO-O-, $-O-CH_2-$, -O-CO- ou une simple liaison ;

k, l, m, n, o, p, q, r valent zéro ou 1, à la condition que la somme de k+m+p+r soit inférieure à 4 et supérieure à zéro.

**2.** 2-Fluoropyrazine selon la revendication 1, caractérisée en ce que les symboles ont les significations suivantes :

$R^1$, $R^2$, indépendamment l'un de l'autre, représentent H, F, CN ou alkyle linéaire ou ramifié avec 1 à 16 atomes de carbone (avec ou sans atome de carbone asymétrique), un groupe $-CH_2-$ pouvant aussi être remplacé par -O-, -S-, -CO-, -CO-O-, -O-CO-, -O-CO-O-, -CH=CH-, -C≡C-,

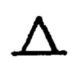

ou $-Si(CH_3)_2-$, ou l'un des groupes chiraux ci-après

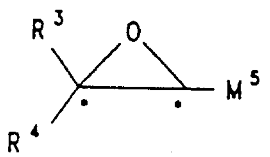 ,
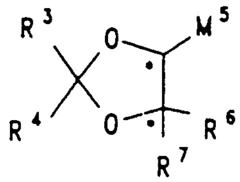 ,
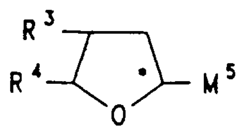 ,

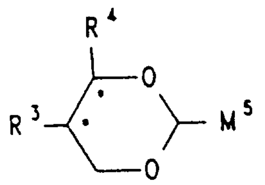 ,
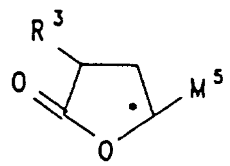 ,

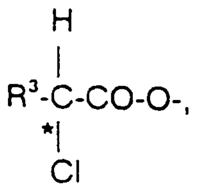
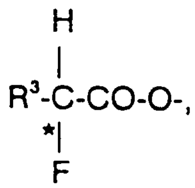
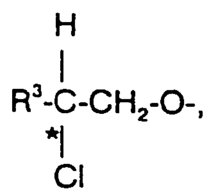
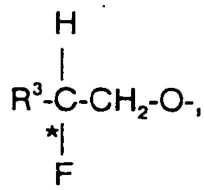

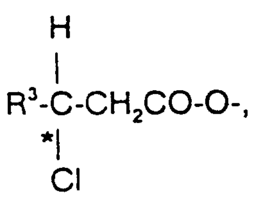
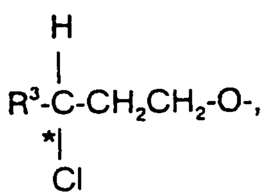

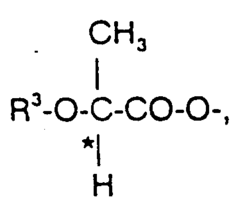
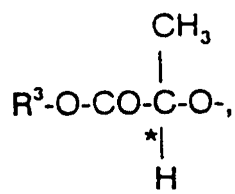

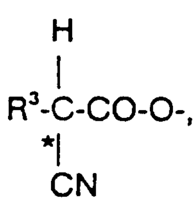
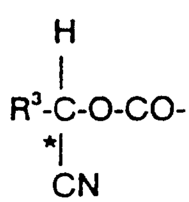

$A^1$, $A^2$, $A^3$, $A^4$, identiques ou différents, représentent 1,4-phénylène, pyrazine-2,5-diyle, pyridazine-3,6-diyle, pyridine-2,5-diyle, pyrimidine-2,5-diyle, un ou deux atomes de H pouvant être remplacés chacun par F, représentent aussi trans-1,4-cyclohexylène, (1,3,4)-thiadiazol-2,5-diyle ou naphtalène-2,6-diyle, 1,3-dioxane-2,5-diyle ou bicyclo[2.2.2.]octane-1,4-diyle ;
$M^1$, $M^2$, $M^3$, $M^4$, identiques ou différents, représentent -O-, -CO-, -CO-O-, -O-CO-, $-CH_2-O-$, $-O-CH_2-$, $-CH_2-$

$CH_2$-, -CH=CH- ou -C≡C- ;

$R^3$, $R^4$, $R^6$, $R^7$, indépendamment les uns des autres, représentent H ou alkyle linéaire ou ramifié avec 1 à 16 atomes de carbone. $R^3$ et $R^4$ ensemble représentent aussi $-(CH_2)_4-$ ou $-(CH_2)_5-$, lorsqu'ils sont liés comme substituants à un système dioxolane ;

$M^5$ représente $-CH_2-O-$, -CO-O-, $-O-CH_2-$, -O-CO- ou une simple liaison.

**3.** 2-Fluoropyrazine selon la revendication 1, caractérisée en ce que les symboles ont les significations suivantes :

$R^1$, $R^2$, indépendamment l'un de l'autre, représentent H, F, CN ou alkyle linéaire ou ramifié avec 1 à 16 atomes de carbone (avec ou sans atome de carbone asymétrique), un groupe $-CH_2-$ pouvant aussi être remplacé par -O-, -CO-, -CO-O-, -O-CO-, -CH=CH-,

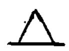

ou $-Si(CH_3)_2-$, ou l'un des groupes chiraux ci-après

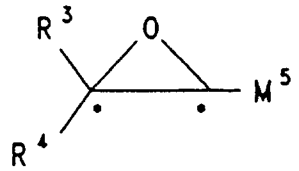 , 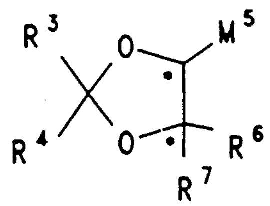 , 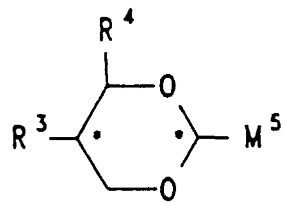 ,

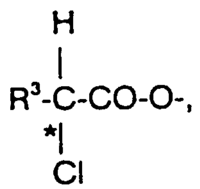

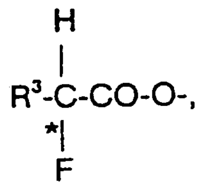

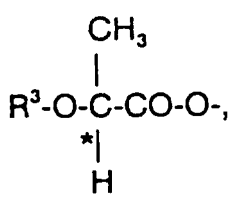

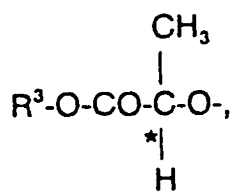

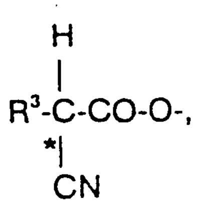

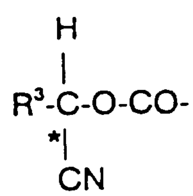

$A^1$, $A^2$, $A^3$, $A^4$, identiques ou différents, représentent 1,4-phénylène, pyrazine-2,5-diyle, pyridazine-3,6-diyle, pyridine-2,5-diyle, pyrimidine-2,5-diyle, où un atome de H pouvant être remplacé par F, représentent aussi trans-1,4-cyclohexylène, naphtalène-2,6-diyle ou 1,3-dioxane-2,5-diyle ;

$M^1$, $M^2$, $M^3$, $M^4$, identiques ou différents, représentent -O-, -CO-O-, -O-CO-, $-O-CH_2-$, $-CH_2-O-$, $-CH_2-CH_2-$ ou -CH=CH- ;

$R^3$, $R^4$, $R^6$, $R^7$, indépendamment les uns des autres, représentent H ou alkyle linéaire ou ramifié avec 1 à 16 atomes de carbone. $R^3$ et $R^4$ ensemble représentent aussi $-(CH_2)_4-$ ou $-(CH_2)_5-$, lorsqu'ils sont liés comme substituants à un système dioxolane ;

$M^5$ représente $-CH_2-O-$, $-CO-O-$, $-O-CH_2-$, $-O-CO-$ ou une simple liaison.

**4.** 2-Fluoropyrazine selon la revendication 1, caractérisée en ce que les symboles dans la formule (I) ont la signification suivante :

$R^1$, $R^2$, indépendamment l'un de l'autre, représentent H ou alkyle avec 1 à 16 atomes de carbone, un groupe $-CH_2-$pouvant être remplacé par -O-, ou représente le groupe chiral

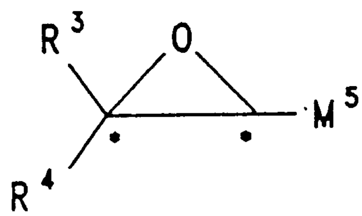

$A^1$, $A^2$, $A^3$, $A^4$, identiques ou différents, représentent 1,4-phénylène, pyrimidine-2,5-diyle, 1,4-cyclohexylène,
$M^1$, $M^2$, $M^3$, $M^4$, identiques ou différents, représentent -O-, -CO-O-, $-CH_2-O-$, $-O-CH_2-$,
$M^5$ représente $-CH_2-O-$, -CO-O- et
$R^3$, $R^4$, indépendamment les uns des autres, représentent H ou alkyle linéaire ou ramifié avec 1 à 16 atomes de carbone.

**5.** Utilisation d'une fluoropyrazine selon la revendication 1 comme composant de mélanges de cristaux liquides.

**6.** Mélange de cristaux liquides composé d'au moins deux composants, caractérisé en ce qu'il contient comme un composant une fluoropyrazine selon la revendication 1.

**7.** Mélange de cristaux liquides ferroélectriques composé de 2 à 20 composants caractérisé en ce qu'il contient comme un composant un composé selon la revendication 1.

**8.** Mélange de cristaux liquides nématiques constitué de 2 à 20 composants caractérisé en ce qu'il contient comme un composant un composé selon la revendication 1.

**9.** Mélange de cristaux liquides ferroélectriques constitué de 2 à 20 composants caractérisé en ce qu'il contient comme un composant un composé selon la revendication 2.

**10.** Mélange de cristaux liquides ferroélectriques constitué de 2 à 20 composants, caractérisé en ce qu'il contient comme un composant un composé selon la revendication 3.

**11.** Mélange de cristaux liquides ferroélectriques constitué de 2 à 20 composants, caractérisé en ce qu'il contient comme un composant un composé selon la revendication 4.

**12.** Mélange de cristaux liquides ferroélectriques constitué de 2 à 20 composants, caractérisé en ce qu'il contient de 1 à 25% en moles d'au moins un composé de formule (I) de la revendication 1.

**13.** Dispositif de commutation et d'affichage ferroélectrique contenant des plaques de support, des électrodes, au moins un polariseur, au moins une couche d'orientation ainsi qu'un milieu à cristaux liquides, caractérisé en ce que le milieu à cristaux liquides est un mélange de cristaux liquides selon la revendication 6.

**14.** Procédé pour la préparation du composé de formule (I) selon la revendication 1, caractérisé en ce que l'on introduit par des réactions en plusieurs étapes les chaînes latérales $R^1(-A^1)_k(-M^1)_l(-A^2)_m-(M^2)_n-$ et $(-M^3)_o(-A^3)_p(-M^4)_q(-A^4)_r-R^2$ en la position 3 ou 6 du cycle pyrazine de la 2,6-dichloropyrazine.

**15.** Procédé selon la revendication 14, caractérisé en ce que l'on fait réagir la 2,6-dichloropyrazine (II) avec un réactif de type fluorure en utilisant des quantités catalytiques d'un agent complexant pour obtenir la 2,6-difluoropyrazine

(III), la 2,6-difluoropyrazine (III) réagit avec un composé métallique de $(-M^3)_o(-A^3)_p(-M^4)_q(-A^4)_r-R^2$ pour obtenir le composé de formule (V) et ensuite, on fait réagir le composé de formule (V) pour obtenir le dérivé de la 2-fluoropyrazine de formule I selon la revendication 1.

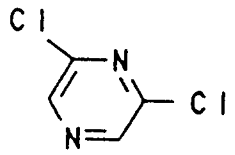
(II)

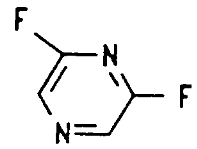
(III)

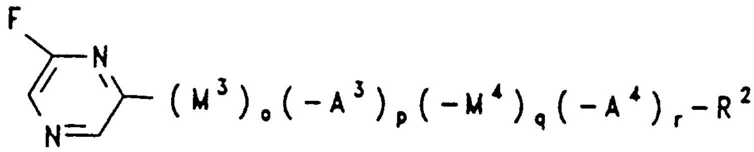
(V)

**16.** Procédé selon la revendication 14, caractérisé en ce que l'on fait réagir la 2,6-dichloropyrazine (II) avec un réactif de type fluorure sous pression réduite, en présence d'un agent complexant pour obtenir la 2-chloro-6-fluoro-pyrazine (IV), la 2-chloro-6-fluoro-pyrazine (IV), avec un dérivé organométallique de $(-M^3)_o(-A^3)_p(-M^4)_q(-A^4)_r-R^2$ en utilisant des catalyseurs à base de métaux de transition pour obtenir le composé de formule (V) et ensuite le dérivé de la 2-fluoropyrazine de formule (I) selon la revendication 1.

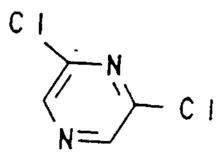
(II)

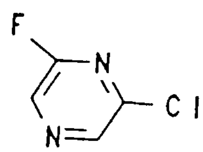
(IV)

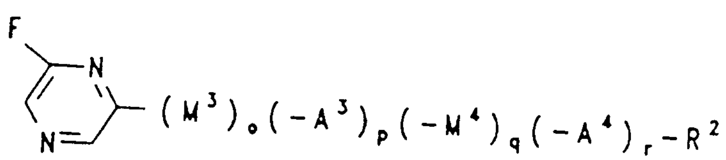
(V)

**17.** Procédé selon la revendication 14, caractérisé en ce que l'on fait réagir un composé de formule (V) en traitant par un composé de lithium pour obtenir le composé de formule (VI),

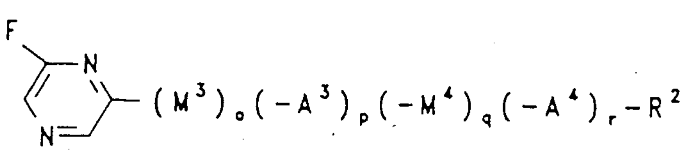
(V)

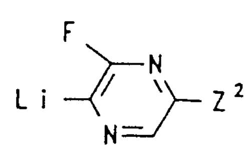
(VI)

et on fait réagir le produit de réaction par un réaction en une ou plusieurs étapes pour obtenir le dérivé de la 2-fluoropyrazine de formule (I) selon la revendication 1.